# EUROPEAN PATENT SPECIFICATION

(11) **EP 2 841 941 B1**
(45) Date of publication and mention of the grant of the patent: **14.02.2018**
(21) Application number: 13720792.4
(22) Date of filing: 26.04.2013
(51) Int. Cl.: C12Q 1/00, G01N 33/50

(54) **METHODS FOR DETERMINING AND/OR MONITORING CONDITIONS OF A THREE DIMENSIONAL CELL CULTURE SYSTEM AND OPTICAL SENSOR DEVICE FOR CONDUCTING SAID METHODS**
VERFAHREN ZUM BESTIMMEN UND/ODER ÜBERWACHEN VON ZUSTÄNDEN EINES DREIDIMENSIONALEN ZELLKULTURSYSTEMS UND OPTISCHE SENSORVORRICHTUNG ZUR DURCHFÜHRUNG DER VERFAHREN
PROCÉDÉS POUR DÉTERMINER ET/OU SURVEILLER LES ÉTATS D'UN SYSTÈME DE CULTURE CELLULAIRE TRIDIMENSIONNELLE ET DISPOSITIF DE CAPTEUR OPTIQUE PERMETTANT DE RÉALISER LESDITS PROCÉDÉS

(30) Priority: 26.04.2012 EP 12165826; 26.04.2012 US 201261638780 P
(43) Date of publication of application: 04.03.2015
(73) Proprietor: TissUse GmbH, 15528 Spreenhagen (DE)
(72) Inventor: MARX, Uwe, 15528 Spreenhagen (DE); KLOKE, Lutz, 10245 Berlin (DE); HORLAND, Reyk, 10627 Berlin (DE); HOFFMANN, Silke, 13187 Berlin (DE); LORENZ, Alexandra, 13055 Berlin (DE); BRINCKER, Sven, 13353 Berlin (DE); SCHIMEK, Katharina, 10555 Berlin (DE)
(74) Representative: Zwicker, Jörk
(86) International application number: PCT/EP2013/001267
(87) International publication number: WO 2013/159939

(56) References cited:
- WO-A1-95/32664
- WO-A1-2008/024855
- WO-A1-2012/016711
- WO-A2-2009/146911
- Sonntag, Frank et al.: "Universelle Geräteplattform für das automatisierte Handlingzellbasierter Assays", 10. Dresdner-Sensor-Symposium 2011 , 6 December 2011 (2011-12-06), XP002679387, Retrieved from the Internet: URL:http://www.google.de/url?sa=t&rct=j&q= multi-organ%20chip%20program%20berlin%20na dh&source=web&cd=2&ved=0CEsQFjAB&url=http% 3A%2F%2Fwww.ama-science.org%2Fhome%2FgetFi le%2FAwH1&ei=55r1T5KAEK_O4QT0qpTpBg&usg=AF QjCNFtMBBfkUBUG7vDbjObV4On9AS6nA&cad=rja [retrieved on 2012-07-05] & "Universelle Geräteplattform für das automatisierte Handling zellbasierter Assays", Posterprogramm, Postersession am Dienstag, 6. Dezember 2011 , 6 December 2011 (2011-12-06), Retrieved from the Internet: URL:http://www.fms-dresden.de/Veranstaltun gen/Sensor_Symposien/10_+Dresdner+Sensor_S ymposium/Posterprogramm-highlight-ger%C3%A 4teplattform-p-57942.html [retrieved on 2012-07-05]
- FRANK SONNTAG ET AL: "Miniaturisierte humane organtypische Zell- und Gewebekulturen", BIOSPEKTRUM, vol. 17, no. 4, 1 June 2011 (2011-06-01), pages 418-421, XP055031986, ISSN: 0947-0867, DOI: 10.1007/s12268-011-0067-6

## Description

The present invention relates to an *in vitro* method for determining and/or monitoring at least one condition of a three dimensional cell culture system comprising at least one growth section, wherein the at least one condition comprises a physiological condition and further comprises a condition selected from the group consisting of vitality, and metabolism status. Also disclosed is an optical sensor device configured to carry out said method.

### BACKGROUND OF THE INVENTION

Up to date, three dimensional cell cultures are still uncommon. They need a continuously sustenance and are, thus, only established in dynamic, e.g. in continuously perfused, bioreactors. Efforts have been made to develop methods for determining and/or monitoring physical conditions, the vitality and the metabolism status of existing three dimensional cell cultures, e.g. organ-on-a-chip (OC) devices, multi-organ-on-a-chip (MOC) devices, or circulation systems. The dimensions of the existing three dimensional cell cultures only support the presence of low volumes of fluid, e.g. the circulation of low volumes of fluid through said systems. This makes the determination or monitoring of physical conditions of said systems difficult.

In addition, in three dimensional cell cultures, the determination or monitoring of the vitality or the metabolic status is complicated in contrast to the observation of cell cultures only comprising a monolayer of cells, particularly with the common optical means, e.g. fluorescence readers.

Moreover, the known optical sensors, e.g. to monitor the metabolic status within said cultures, do not work contact-free and non-invasive. For example, said sensors have to be introduced or incorporated in the three dimensional cultures so that the direct intervention with the environment of the three dimensional cell cultures systems cannot be avoided. This can lead to the contamination or disturbance of the sensible or sterile atmosphere in said cultures which is not desirable.

Thus, there is a need for new methods which allow the determination or monitoring of physical conditions, the vitality and the metabolism status of three dimensional cell cultures and for new optical sensors which can be used in these methods.

The inventors of the present invention have developed new methods which allow the determination of physical conditions of three dimensional cell cultures on the basis of erythrocytes as detectors. The use of erythrocytes as detectors has the advantage that no mechanical measurement is required. That means that no foreign matter has to be introduced in the three dimensional cell cultures which may contaminate or disturb the sensible and/or sterile atmosphere therein.

Further, the inventors of the present invention have developed methods which allow the determination of the vitality and metabolism status in said cultures.

Furthermore, the inventors of the present invention have developed an optical sensor which is configured to allow the contact-free and non-invasive conduction of the methods for determining and/or monitoring physical conditions, the vitality and the metabolism status of three dimensional cell cultures. The use of said optical sensor, thus, avoids the direct intervention with the sterile and/or sensible environment of said cultures. Said optical sensor is further configured to allow the conduction of the above methods in three dimensional cell cultures and not only in a monolayer of cells.

### BRIEF SUMMARY OF THE INVENTION

The present invention relates to an *in vitro* method for determining and/or monitoring at least one condition of/in a three dimensional cell culture system (2) comprising at least one growth section (3), wherein the at least one condition comprises a physiological condition and further comprises a condition selected from the group consisting of
(i) vitality, and
(ii) metabolism status,
wherein the physiological condition is determined using erythrocytes as detectors for said condition by determining the flow rate, physiological osmolality, and/or oxygen consumption in the three dimensional cell culture system (2), the vitality is determined and/or monitored by measuring living cell fluorescent dyes, and the metabolism status is determined and/or monitored by measuring the autofluorescence of NADH and/or FAD, and/or by determining the NADH/NAD⁺ and/or NADH/FAD ratio.

Also disclosed is an optical sensor device (1) configured to carry out the method for determining and/or monitoring at least one condition in a three dimensional cell culture system (2) comprising at least one growth section (3), wherein the at least one condition is selected from the group consisting of
(i) physiological condition,
(ii) vitality, and
(iii) metabolism status,
wherein the physiological condition is determined using erythrocytes as detectors for said condition, the vitality is determined and/or monitored by measuring living cell fluorescent 2 dyes, and the metabolism status is determined and/or monitored by measuring the autofluorescence of NADH and/or FAD, and/or by determining the NADH/NAD⁺ and/or NADH/FAD ratio according to the present invention.

Also disclosed is the use of the optical sensor device (1) disclosed herein for determining and/or monitoring at least one condition in a three dimensional cell culture system (2) comprising at least one growth section (3), wherein the at least one condition is selected from the group consisting of
(i) physiological condition,
(ii) vitality, and
(iii) metabolism status,
wherein the physiological condition is determined using erythrocytes as detectors for said condition, the vitality is determined and/or monitored by measuring living cell fluorescent dyes, and the metabolism status is determined and/or monitored by measuring the autofluorescence of NADH and/or FAD, and/or by determining the NADH/NAD⁺ and/or NADH/FAD ratio.

Also disclosed is the use of the optical sensor device (1) disclosed herein for monitoring the effect of a test compound and/or for determining the efficacy, side-effects, biosafety, metabolites, mode of action or organ regeneration.

This summary of the invention does not necessarily describe all features of the invention.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Klbl, H. eds. (1995), Helvetica Chimica Acta, CH-4010 Basel, Switzerland) and as described in "Pharmaceutical Substances: Syntheses, Patents, Applications" by Axel Kleemann and Jurgen Engel, Thieme Medical Publishing, 1999; the "Merck Index: An Encyclopedia of Chemicals, Drugs, and Biologicals", edited by Susan Budavari et al., CRC Press, 1996, and the United States Pharmacopeia-25/National Formulary-20, published by the United States Pharmcopeial Convention, Inc., Rockville Md., 2001.

Throughout this specification and the claims which follow, unless the context requires otherwise, the word "comprise", and variations such as "comprises" and "comprising", will be understood to imply the inclusion of a stated feature, integer or step or group of features, integers or steps but not the exclusion of any other feature, integer or step or group of integers or steps. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

The term "about" when used in connection with a numerical value is meant to encompass numerical values within a range having a lower limit that is 5% smaller than the indicated numerical value and having an upper limit that is 5% larger than the indicated numerical value.

Several documents are cited throughout the text of this specification. Nothing herein is to be construed as an admission that the invention is not entitled to antedate such disclosure by virtue of prior invention.

In the following, some definitions of terms frequently used in this specification are provided. These terms will, in each instance of its use, in the remainder of the specification have the respectively defined meaning and preferred meanings:
The term "cells", as used herein, means cell lines or primary cells of vertebrates or invertebrates.

The term "organoids", as used herein, means artificial, *de novo* generated, functional cell aggregates of different types of cells *in vitro* that show at least one organ or tissue function, preferably shows the majority of or essentially all organ or tissue functions.

The term "organ", as used herein, means artificial, *de novo* generated, functional cell aggregates of different types of cells *in vitro* that show all functions of the natural organ.

The term "tissues", as used herein, stands for biopsy material or explants taken from patients or animals or in vitro generated tissues.

The term "cell growth", as used herein, refers to the growth of cell populations, where one cell (the "mother cell") grows and divides to produce two "daughter cells" (M phase) and refers to the increase in cytoplasmic and organelle volume (G1 phase), as well as increase in genetic material before replication (G2 phase). During cell growth and differentiation,

The term "differentiation", as used herein, means the development of tissue specific functions of cultured cells.

The term "maintenance", as used herein, describes the ability to keep all functions of a given tissue constant within a given cell culture process, preferably without significant signs of cell death and/or apoptosis .

The term "medium" (plural form: "media"), as used herein, means growth supporting liquid with nutrients and substances for cultivation of cells. Examples of suitable media comprise DMEM, Ham's F12 and RPMI.

The term "supplements", as used herein, describe substances to be added to culture media in order to induce or modify cell function, which may have a defined composition like, e.g. purified or recombinant cytokines or growth factors, or which are undefined like, e.g. serum.

The term "matrix", as used herein, means substances or mixtures of substances, which maintain viability, enhance proliferation, differentiation, and function of cells, cell aggregates, tissues, organoids and/or organs. Matrix material is preferably provided in a form which can be fitted to fill the space of the growth section (3). Matrixes usable in the context of the present invention can take a variety of shapes comprising, e.g. hydrogels, foams, fabrics or non-woven fabrics. The matrix material may comprise naturally occurring matrix substances like extracellular matrix proteins, preferably collagens, laminins, elastin, vitronectin, fibronectin, small matricellular proteins, small integrin-binding glycoproteins, growth factors or proteoglycans or may include artificial matrix substances like non-degradable polymers such as polyamid fibres, methylcellulose, agarose or alginate gels or degradable polymers, e.g. polylactid.

To overcome the problems associated with prior art, the present invention provides an *in vitro* method for determining and/or monitoring at least one condition, preferably two or three conditions, of a three dimensional cell culture system (2) comprising or consisting of at least one growth section (3) or in a three dimensional cell culture system (2) comprising or consisting of at least one growth section (3), wherein the at least one condition, preferably two or three conditions, comprise(s) a physiological condition and further comprises a condition selected from the group consisting of
(i) vitality, and
(ii) metabolism status,
wherein the physiological condition is determined using erythrocytes as detectors for said condition by determining the flow rate, physiological osmolality, and/or oxygen consumption in the three dimensional cell culture system (2), the vitality is determined and/or monitored by measuring living cell fluorescent dyes, and the metabolism status is determined and/or monitored by measuring the autofluorescence of NADH and/or FAD, and/or by determining the NADH/NAD⁺ and/or NADH/FAD ratio.

The above method for determining and/or monitoring the above mentioned at least one condition, e.g. at least one, two, or three condition(s), is preferably carried out contact-free and non-invasive, particularly to avoid the direct intervention with the environment of the three dimensional cell culture system (2), e.g. the sterile, self-contained, and/or circulation three dimensional cell culture system (2). In addition, said method does not require the manipulation and/or modification of the three dimensional cell culture system (2), e.g. the sterile, self-contained, and/or circulation three dimensional cell culture system (2). The contact-free and non-invasive determination and/or monitoring of the at least one condition, e.g. at least one, two, or three condition(s), which does not require the manipulation and/or modification of the three dimensional cell culture system (2), is particularly conducted using optical means, particularly using the optical sensor device (1) disclosed herein. In this respect, it is further preferred that the three dimensional cell culture system (2), preferably the body of the three dimensional cell culture system (2), is made of a translucent material, e.g. of glass, such as calcium carbonate/sodium bicarbonate glass or quartz glass, or plastic. This allows the contact-free and non-invasive observation of the three dimensional cell culture system (2), particularly using optical means, more particularly using the optical sensor device (1) disclosed herein. Particularly, it allows the analysis of the three dimensional cell culture system (2) from the outside.

The term "three dimensional cell culture system (2)", as used herein, refers to a three dimensional (3D) assembly of cells which are cultured. The term "cell culture" refers to a complex process which allows the growth, the differentiation and/or the maintenance of cells under controlled conditions, generally outside of their natural environment. Said three dimensional assembly of cells is preferably made from multiple individually structured and/or microstructured cell layers that are in fluid-tight connection with each other and is particularly capable to provide a fluid-tight environment and, thus, preferably sterile environment. Said three dimensional assembly of cells preferably refers to a multilayer cell assembly, a cell aggregate, a tissue, an organoid, or an organ. During culture, the multilayer cell assembly or cell aggregate may further develop, e.g. by cell growth and/or differentiation, to an organoid or organ. The three dimensional cell culture system (2) is preferably dimensioned to be used in standard high throughput set ups, e.g. having the size of a standard microtiterplate or strip. Thus, preferably the width is between 2 to 10 cm, preferably 1, 2, 3, 4, 5, 6, 7, 8, 9, or 10 cm and/or the length between 3 and 15 cm, preferably 3, 4, 5, 6, 7, 8, 9, 10, 11, 1, 12, 13, 14 or 15 cm and/or the height between 0.2 and 10 mm, more preferably between 1 and 4 mm, i.e. 0.2, 0.3, 0.4, 0.5, 0.6, 0.7, 0.8, 0.9, 1.0, 1.1, 1.2, 1.3, 1.4, 1.5, 1.6, 1.7, 1,8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 5.0, 6.0, 7.0, 8.0, 9.0 or 10.0 mm. To conform to the standard microtiterplate format the width to length are preferably in a ratio of about 1:3. Particularly preferred is a size of 2.5 cm width, 7.5 cm length and 3 mm height.

Preferred materials comprise SiO₂, glass, and synthetic polymers. Preferred synthetic polymers comprise polystyrol (PS), polycarbonate (PC), polyamide (PA), polyimide (PI), polyetheretherketone (PEEK), polyphenylenesulfide (PPSE), epoxide resin (EP), unsaturated polyester (UP), phenol resin (PF), polysiloxane, e.g. polydimethylsiloxane (PDMS), melamine resin (MF), cyanate ester (CA), polytetrafluoroethylene (PTFE) and mixtures thereof. Particularly preferred synthetic polymers are optically transparent and include, e.g. polystyrol (PS), polycarbonate (PC), and polysiloxane, e.g. polydimethylsiloxane (PDMS).

In its simplest form, the three dimensional cell culture system (2) consists of at least one growth section (3). Such a three dimensional cell culture system (2) may have the form of a cell culture tube or cell culture well. It is, however, particularly preferred that the growth section (3) is a microstructured region which is comprised within a three dimensional cell culture system (2).

The term "growth section (3)", as used herein, refers to a region which provides the entire micro-environment for cell, cell aggregate, tissue, organoid and/or organ growth, differentiation, and/or maintenance. The growth section (3) preferably includes a micro inlet, e.g. a medium and/or blood inlet, and/or a micro outlet, e.g. medium and/or blood outlet, that holds the majority of the cells forming the respective cell aggregates, tissues, organoids and/or organs, and/or an open surface, which may be covered in an essentially fluid-tight and gas permeable or fluid-tight and gas permeable way by appropriate means, including a membrane, e.g. PTFE membranes, fibrin sheets, spray-on band aid sheets and/or sheets of coagulation products, once the cells, cell aggregates, tissues, organoids, and/or organs have been loaded into the growth section (3) or by flexible sheets that cover the opening, e.g. lips made from flexible material like polysiloxane, e.g. PDMS. In a preferred embodiment such flexible sheet covers the entire growth section and has cuts allowing access through the cut to the individual cells, cell aggregates, tissues, organoids, and/or organs. The flexible sheets have the advantage that the growth section remains accessible without the necessity to reseal the membrane after access. Preferably the covered surface is fluid-tight but gas permeable and, thus, allows exchange of O₂ and CO₂ between the cells in the growth section (3) and the environment. Preferably the growth section (3) has an essentially circular or a circular form, e.g. the form of a flat cylinder. The ratio of diameter to height of a growth section (3) is preferably between 2:1 to 6:1, more preferably between 3:1 to 5:1. Preferably, the growth section (3) has a surface of 0.1. to 3 cm² preferably 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0 cm², particularly preferred growth sections (3) have a surface area of between 0.3 to 0.7 cm², preferably 0.56 cm². If the growth section (3) has a circular shape it is preferred that it has a diameter of between 0.1 and 1 cm, preferably 0.1, 0.2, 0.3, 0.4, 0.5, 0.6, 0.7, 0.8, 0.9, or 1.0, most preferably 0.6 cm. The three dimensional cell culture system (2) preferably comprises or consists of more than one growth section (3). Given the indicated preferred sizes of each growth section (3) it is possible to fit large numbers of separate growth sections (3) on one three dimensional cell culture system (2). Preferably, one three dimensional cell culture system (2) comprises or consists of between 2 and 2000 growth sections (3), e.g. 2, 3, 4, 5, 6, 7, 8, 9, 10, 11, 12, 13, 14, 15, 16, 17, 18, 19, 20, 24, 30, 36, 48, 60, 72, 84, 96, 100, 108, 120, 132, 144, 156, 168, 180, 192, 204, 216, 228, 240, or more growth sections (3). In a preferred microtiterplate-like format, 2, 4, 6, 24, 96, 384 or even 1536 growth sections (3) are arranged in a 2:3 rectangular matrix on the three dimensional cell culture system (2).

The at least one growth section (3) is preferably filled with a matrix which provides a growth scaffold for the capillaries which are formed or which will be formed by growing cells, e.g. endothelial cells, in the growth section (3). As to the definition of the term "matrix" it is referred to the above. It is, thus, preferred that the matrix comprises micro channels, structures and/or networks, which allow and support formation of capillaries by cells, e.g. by endothelial cells. Preferably, these structures themselves do not have the shape of the later capillaries but merely provide attachment points and/or guidance for the capillaries formed. It is preferred that the growth section (3) comprises or consists of a semi-solid, biodegradable, and/or biocompatible matrix. Alternatively, biocompatible hollow fibres are provided either alone or embedded in a matrix as set out above. The hollow fibres, micro channels, structures, and/or networks are preferably connected at one side to the micro inlet and at the other side to the micro outlet, thereby guiding the growth of the cells, e.g. endothelial cells.

The growth section (3) preferably comprises a cavity termed "growth cavity" which holds the majority of the cells, i.e. at least 80%, preferably 85%, 90%, 95%, 98% or more of the cells comprised in the growth section (3). The growth cavity preferably has the proper dimension, shape and nutrition for each specific cell aggregate, tissue, organoid, and/or organ. It preferably provides access to introduce additionally necessary elements of micro-architecture and micro-environment and/or to load the three dimensional cell culture system (2) with the cell suspension, cell clusters and/or tissue slices, as the case may be. It is preferably coated with appropriate materials and/or contains matrixes to guide/attract/maintain cells.

The growth section (3) preferably further comprises an extra-capillary fluid and/or waste collector/reservoir. The drainage of extra capillary fluid is driven by intra-capillary pressure differences. This serves the purpose of draining fluids away from the cell aggregates, tissues, organoids and/or organs, e.g. pancreas, kidney, gut, which secrete fluids extra capillary. Preferably, the waste collector is separated from the above described matrix and/or hollow fibres in a way, which prevents the efflux of blood cells and/or tissue or organoid cells from the growth section (3). Preferably the extra-capillary fluid and/or waste collector is separated from the remaining growth section (3) by a cell retention membrane, i.e. a membrane having an average pore size smaller than the average size of the cells growing in the three dimensional cell culture system (2) or by cell exclusion channels, which are sized to exclude the cells growing in the three dimensional cell culture system (2).

It is preferred that the three dimensional cell culture system (2) comprises two or more growth sections (3), preferably comprising a growth cavity, so that each of the growth sections (3) can provide the appropriate microenvironment for a different cell aggregate, tissue, organoid and/or organ, e.g. one growth section for neurons, one growth section for heart tissue, one growth section for cartilage, one growth section for bone and/or one growth section for vascularised skin. In this way it is, for example, possible to assess the effect of one particular test compound on several/different cell aggregates, tissues, organoids and/or organs simultaneously. Alternatively, the three dimensional cell culture system (2) may comprise two or more growth sections (3), preferably comprising a growth cavity, providing the appropriate microenvironment for the same type of cell aggregate, tissue, organoid and/or organ the same type, e.g. growth sections for neurons, heart tissue, cartilage, bone or vascular skin. This allows, for example, the determination of the effect of a given test compound with a higher statistical significance by averaging the results obtained from two, three, four or more growth sections (3) in parallel. In addition, one growth section (3), preferably comprising a growth cavity, may comprise cells of a particular cell type, which may serve as a standard, for each measurement.

A growth cavity within a growth section (3) has typically a volume between 1 x 10² to 0.01 mm³, preferably 100, 90, 80, 70, 60, 50, 40, 30, 20, 10, 9, 8, 7, 6, 5, 4, 3, 2, 1, 0.9, 0.8, 0.7, 0.6, 0.5, 0.4, 0.3, 0.2, 0.1, 0.09, 0.08, 0.07, 0.06 and 0.05 mm³, preferably 1 mm³.

It is preferred that the three dimensional cell culture system (2) is a system which is self-contained. It is also preferred that the three dimensional cell culture system (2) is a circulation system. More preferably, the three dimensional cell culture system (2) is a self-contained and circulation system.

The term "self-contained" refers to the fact that media, blood and supplements required for differentiation and maintenance of cells, cell aggregates, tissues, organoids, and/or organs in the at least one growth section (3) are provided from the three dimensional cell culture system (2). For this purpose, at least one fluid reservoir such as medium and/or blood reservoir is preferably comprised within the three dimensional cell culture system (2). The at least one fluid reservoir such as medium and/or blood reservoir is preferably connected through at least one microfluidic channel within the three dimensional cell culture system (2) to the at least one growth section (3). Thus, there is no fluidic connection providing fluid from an external fluid reservoir. Accordingly, the self-contained three dimensional cell culture system (2) can be handled and moved, without the danger of contaminating the medium, blood and subsequently the cells, cell aggregates, tissues, organoids, and/or organs within the at least one growth section (3). Additionally, it is preferred that gaseous medium, e.g. O₂/CO₂, is provided to the at least one growth section (3) in a passive manner, i.e. by diffusion into the medium through a membrane or biocompatible polymer foil from the environment. This membrane or polymer foil is preferably fluid-tight. Again this is preferred to allow the handling of the three dimensional cell culture system (2). Preferably, the membrane or foil covers at least partially, more preferably fully, the growth section (3), thus allowing O₂/CO₂ to diffuse into the fluid such as medium or blood flowing through the microfluidic channels and/or through the at least one growth section (3). In a preferred embodiment, the membrane is formed or attached after cells, cell aggregates, tissues, organoids and/or organs have been loaded into the growth section (3) or it forms an integral part of the three dimensional cell culture system (2). Accordingly, in a preferred embodiment the three dimensional cell culture system (2) comprises no connectors to an external gaseous medium supply and/or does not comprise a device for actively aerating the medium.

The flow of fluids through the microfluidic system of the self-contained three dimensional cell culture system (2) may be achieved by gravity or capillary forces. To ascertain the flow of medium through the system it is, however, preferred that the medium or blood reservoir and/or the waste reservoir comprises a hydrophilic material, which - one wetted - absorbs the medium and, thus, provides a suction that is suitable to provide a fluid flow. Alternatively, a micro-pump may be arranged in the three dimensional cell culture system (2), e.g. between the at least one medium and/or blood reservoir and between the at least one growth section (3).

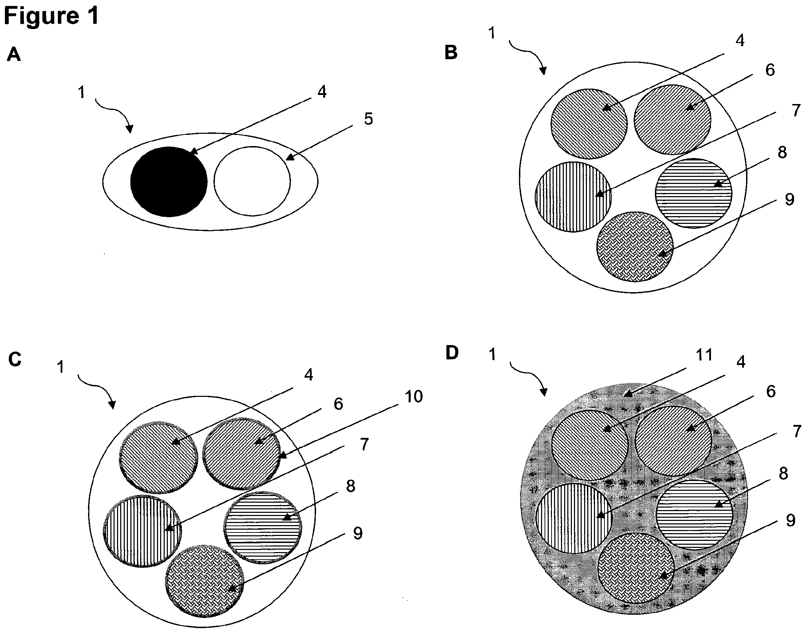

A preferred three dimensional cell culture system (2), preferably "self-contained" three dimensional cell culture system (2), is described in WO 2009/146911 A2 on page 2, line 22 to page 3, line 4, page 11, line 25 to page 36, line 35, and page 38, line 24 to page 39, line 15, and in claims 1 to 32, and is shown in Figures 1 to 8 (see Figure legend on page 4, line 4 to page 9, line 3) of WO 2009/146911 A2. A method to produce such a preferred "self-contained" three dimensional cell culture system (2) is further described in WO 2009/146911 A2 on page 3, lines 6 to 20, page 37, page 1 to page 38, line 2, and page 38, line 24 to page 39, line 15, and in claims 33, and 38 to 42.

The term "circulation" refers to the fact that fluid, e.g. medium and/or blood, in the three dimensional cell culture system (2) is circulated within said cell culture system. A preferred circulation three dimensional cell culture system (2) comprises at least one growth section (3) and at least one directional pumping device. In such a system, the fluid, e.g. medium and/or blood, is preferably circulated between the at least one growth section (3) and the at least one directional pumping device, particularly via one or more microfluidic channels. It is preferred that the fluid, e.g. medium and/or blood, is circulated within the above-described "self-contained" three dimensional cell culture system (2). In this case, fluid, e.g. medium and/or blood, is preferably circulated between the at least one fluid reservoir such as medium and/or blood reservoir and the at least one growth section (3), particularly via one or more microfluidic channels. Such a "self-contained" "circulation" three dimensional cell culture system (2) preferably further comprises at least one directional pumping device. In this case, the fluid in the "self contained" "circulation" three dimensional cell culture system (2) is circulated between the at least one directional pumping device, the at least one growth section (3) and the at least one fluid reservoir such as medium and/or blood reservoir, particularly via one or more microfluidic channels.

A preferred "self-contained" "circulation" three dimensional cell culture system (2) is described in WO 2012/016711 A1 on page 3, lines 6 to 13, page 5, line 20 to page 14, line 34, page 16, line 19 to page 19, line 9, and page 20, lines 5 to 23, and in claims 1 to 17 and is shown in Figures 1 to 5 (see Figure legend on page 20, line 24 to page 21, line 31) of WO 2012/016711 A1. A method to produce such a preferred "self-contained" "circulation" three dimensional cell culture system (2) is further described in WO 2012/016711 A1 on page 3, lines 14 to 21, on page 15, line 1 to page 16, line 14, page 19, lines 10 to 33, page 20, lines 5 to 23, and page 22, line 1 to page 23, line 8 and in claims 18 to 20.

During operation of the three dimensional cell culture system (2), the two, three, four, five or more identical or different cell aggregates, tissues, organoids, or organs that are formed or maintained separately in the two, three, four, five or more growth sections (3), preferably in the two, three, four, five or more growth cavities comprised in said growth sections (3) may interact with each other. Interaction may occur between the growth sections (3), preferably between the growth cavities, through, e.g. outgrowth of nerves and/or microcapillaries from one growth section (3), particularly growth cavity, to another growth section (3), particularly growth cavity. Such interaction may occur through separately provided connecting channels and/or openings between the different growth sections (3), particularly growth cavities. Said connecting channels and/or openings may be opened or closed as desired.

While it is possible to assess several properties of the cell aggregates, tissues, organoids, and/or organs comprised in the at least one growth section (3), particularly in the at least one growth cavity comprised therein, by optical means, particularly with an optical sensor, more particularly with an optical sensor device (1) disclosed herein, it is preferred that the three dimensional cell culture system (2), e.g. the self-contained and/or circulation three dimensional cell culture system (2), is microscopable. To the end every part of the three dimensional cell culture system (2) may be manufactured from an optically transparent material. Preferably, the at least one growth section (3), the at least one growth cavity comprised therein, the at least one microfluidic channel, the fluid reservoir, e.g. blood and/or medium reservoir, and/or waste reservoir are microscopable.

The medium or blood reservoir which may be comprised in the self-contained three dimensional cell culture system (2) holds the medium, blood and/or supplements necessary to differentiate and/or maintain the cell aggregates, tissues, organoids, or organs in the at least one growth section (3). The size of the medium or blood reservoir comprised in the self-contained three dimensional cell culture system (2) is determined by several parameters including: (i) required self-contained cultivation period and (ii) required medium change rate. Typically the medium or blood reservoir comprises medium in excess of one growth cavity volume per day of culture multiplied by the number of culture days and, if required supplements. In a preferred embodiment the self-contained cultivation period is at least 3 days, 7 days, 14 days, 21 days, 28 days, 90 days 180 days, 365 days, or more. In a typical embodiment the medium or blood reservoir comprised within the self-contained organ-on-a-chip device has a volume of between 5 µl and 5000 µl, preferably a volume of between 20 µl and 200 µl.

The blood reservoir comprises blood, particularly whole blood or a blood fraction such as plasma, serum, or blood cells (also known as hemopoietic cell), e.g. from vertebrates such as mammals like humans, or invertebrates. The term "hemopoietic cells" refers to mature cell types and their immature precursors that are identifiable either by morphology or, mostly, by a distinct pattern of cell surface markers. The term is used to distinguish these cells from other cell types found in the body and also includes T-cells and distinctive subsets, which are the only hematopoietic cells that are not generated in the bone marrow. Preferably, the blood cells are erythrocytes, leukocytes and/or thrombocytes. More preferably, the blood reservoir comprises whole blood or blood plasma (both comprising erythrocytes). The blood cells, e.g. erythrocytes, may also be comprised in the medium which is comprised in the medium reservoir. The use of blood, preferably whole blood, ascertains a strong buffer system, provides all necessary proteins of the plasma to the tissues, supports oxygen transport through the erythrocytes and provides immunological activities against contaminating microorganisms through white blood cells.

As mentioned above, the self-contained and/or circulation three dimensional cell culture system (2) may comprise at least one microfluidic channel. Preferably, said at least one microfluidic channel fluidically connects the least one medium or blood reservoir with the at least one growth section (3), fluidically connects the at least one directional pumping device with the at least one growth section (3) or fluidically connects the at least one medium or blood reservoir, the at least one growth section (3) and the at least one directional pumping device with each other. The diameter of the microfluidic channels is preferably between 100 nm to 1 mm, preferably between 0.5 µm to 200 µm, more preferably 1 µm to 100 µm. It is preferred that the microfluidic channel is provided with a further outlet, which allows the administration of supplements and/or test compounds to the growth sections (3) separately.

It is preferred that the directional pumping device is a biological pump, hydraulic pump, piezoelectric pump peristaltic pump, pneumatic pump, electro-magnetic pump or magnetic pump. A biological pump is formed, e.g. by cardiomyocytes, which are seeded preferably on elastic polymers of a shape supporting pulsate flow at cardiomyocyte contraction (Tanaka et al., 2006, Lab Chip, 6, 362-386). The twitching of the cardiomyocytes provides the contraction necessary for the pumping action.

The three-dimensional cell culture system (2) may further comprise an injection and/or a rejection port. The term "injection port" refers to an opening which allows the injection of material, e.g. substances such as nutrients, fluids such as media and/or blood, test compounds, chemicals such as living fluorescent dyes. The term "rejection port" refers to an opening which allows the rejection of material, e.g. fluids such as media and/or blood. In preferred embodiments, the port is configured to allow both, the injection and rejection of material, e.g. in order to remove/exchange the fluids, e.g. media and/or blood, comprised in the three dimensional cell culture system (2). The injection and/or rejection port is preferably located in a microfluidic transport channel or connected to the at least one growth section (3) of the three dimensional cell culture system (2). For example, if substances, e.g. nutrients, fluids such as media and/or blood, test compounds, chemicals such as living fluorescent dyes, have to be replenished or added during the course of incubation to the three dimensional cell culture system (2), it is preferred that such substances are supplied discontinuously through an injection port, which is preferably located in a microfluidic transport channel or connected to the at least one growth section (3).

Preferably, the dimensions of the "circulation" three dimensional cell culture system (2) support the continuous circulation of between 5 and 5000 µl, more preferably of between 20 and 200 µl, of blood or medium through said system. The above described "self-contained" and/or "circulation" three dimensional cell culture system (2) preferably allow for continuous nutrient supply and waste removal.

As mentioned above, the present invention relates to an *in vitro* method for determining and/or monitoring at least one condition, preferably two or three conditions, of a three dimensional cell culture system (2) comprising or consisting of at least one growth section (3) or in a three dimensional cell culture system (2) comprising or consisting of at least one growth section (3), wherein the at least one condition, preferably two or three conditions, comprise(s) a physiological condition and further comprises a condition selected from the group consisting of
(i) vitality, and
(ii) metabolism status,
wherein the physiological condition is determined using erythrocytes as detectors for said condition by determining the flow rate, physiological osmolality, and/or oxygen consumption in the three dimensional cell culture system (2), the vitality is determined and/or monitored by measuring living cell fluorescent dyes, and the metabolism status is determined and/or monitored by measuring the autofluorescence of NADH (NADH = reduced form of nicotinamide adenine dinucleotide) and/or FAD (FAD = oxidised form of flavin adenine dinucleotide), and/or by determining the NADH/NAD⁺ (NAD⁺ = oxidised form of nicotinamide adenine dinucleotide) and/or NADH/FAD ratio.

Preferably, the (two or three) conditions determined and/or monitored are (i) the physiological condition and the vitality, (ii) the physiological condition and the metabolism status, or (iiii) the physiological condition, the vitality and the metabolism status, wherein the physiological condition is determined using erythrocytes as detectors for said condition, the vitality is determined and/or monitored by measuring living cell fluorescent dyes, and the metabolism status is determined and/or monitored by measuring the autofluorescence of NADH and/or FAD, and/or by determining the NADH/NAD⁺ and/or NADH/FAD ratio.

The term "cell vitality", as used herein, means the aliveness of cells characterized by the capacity to perform certain essential vital functions such as growth, reproduction, some form of responsiveness, and adaptability. In the method of the present invention, the vitality of the three dimensional cell culture system (2), particularly of the multilayer cell assembly/assemblies, cell aggregate(s), tissue(s), organoid(s), and/or organ(s) comprised in the three dimensional cell culture system (2), is determined and/or monitored by measuring living cell fluorescent dyes.

The term "cell metabolism", as used herein, means the total of all the chemical processes that occur in living cells resulting in growth, production of energy, elimination of waste material and detoxification of external substances. In the method of the present invention, the metabolism status of the three dimensional cell culture system (2), particularly of the multilayer cell assembly/assemblies, cell aggregate(s), tissue(s), organoid(s), and/or organ(s) comprised in the three dimensional cell culture system (2), is determined and/or monitored by measuring the autofluorescence of NADH and/or FAD, and/or by determining the NADH/NAD⁺ and/or NADH/FAD ratio.

The term "physiological condition", as used herein, refers to a condition of the internal milieu of cells, e.g. pH, oxygen concentration, and/or glucose concentration. The physiological condition is preferably the flow rate, physiological osmolality, and/or oxygen consumption in the three dimensional cell culture system (2) or of the three dimensional cell culture system (2), e.g. the flow rate of fluids, such as blood and/or plasma, flowing through, circulating in, or perfusing the three dimensional cell culture system (2), the physiological osmolality of the fluids, such as blood and/or plasma, comprised in the three dimensional cell culture system, particularly perfusing the multilayer cell assembly/assemblies, cell aggregate(s), tissue(s), organoid(s), and/or organ(s) comprised in the at least one growth section (3) of the three dimensional cell culture system (2), and/or the oxygen consumption of the multilayer cell assembly/assemblies, cell aggregate(s), tissue(s), organoid(s), and/or organ(s) comprised in the at least one growth section (3) of the three dimensional cell culture system (2).

In the method of the present invention, the physiological condition in the three dimensional cell culture system (2) or of the three dimensional cell culture system (2), particularly of the multilayer cell assembly/assemblies, cell aggregate(s), tissue(s), organoid(s), and/or organ(s) comprised in the at least one growth section (3) of the three dimensional cell culture system (2), is determined using erythrocytes as detectors for said condition. According to the present invention erythrocytes are used as detectors for determining the flow rate, physiological osmolality, and/or oxygen consumption in the three dimensional cell culture system (2) or of the three dimensional cell culture system (2), e.g. (i) the flow rate and physiological osmolality, (ii) the flow rate and oxygen consumption, (iii) the physiological osmolality and the oxygen consumption, or (iv) the flow rate, physiological osmolality and oxygen consumption. For example, erythrocytes may be used as detectors for determining the flow rate of fluids, such as blood and/or plasma, flowing through, circulating in, or perfusing the three dimensional cell culture system (2), the physiological osmolality of the fluids, such as blood and/or plasma, comprised in the three dimensional cell culture system, particularly perfusing the multilayer cell assembly/assemblies, cell aggregate(s), tissue(s), organoid(s), and/or organ(s) comprised in the at least one growth section (3) of the three dimensional cell culture system (2), and/or the oxygen consumption of the multilayer cell assembly/assemblies, cell aggregate(s), tissue(s), organoid(s), and/or organ(s) comprised in the at least one growth section (3) of the three dimensional cell culture system (2).

In a preferred embodiment, the method for determining the flow rate in the three dimensional cell culture system (2) using erythrocytes as detectors comprises the steps of:
(i) providing a perfusable three dimensional cell culture system (2) comprising at least one growth section (3),
(ii) taking pictures of erythrocytes in said cell culture system at least at two different time points, e.g. 2, 3, or 4 different time points (and thereby obtaining at least two pictures, e.g. 2, 3, or 4 pictures),
(iii) selecting at least one erythrocyte, e.g. 1, 2, 3, 4, 5, 6, 7, 8, 9, 10, 50, 100 erythrocyte(s), and determining the position of the at least one erythrocyte, e.g. 1, 2, 3, 4, 5, 6, 7, 8, 9, 10, 50, 100 erythrocyte(s), on the at least two pictures, e.g. 2, 3, or 4 pictures,
(iv) assigning a motion vector to the at least one erythrocyte, e.g. 1, 2, 3, 4, 5, 6, 7, 8, 9, 10, 50, 100 erythrocyte(s), representing the positional change of said at least one erythrocyte, e.g. 1, 2, 3, 4, 5, 6, 7, 8, 9, 10, 50, 100 erythrocyte(s), on the at least two pictures, e.g. 2, 3, or 4 pictures, and
(v) determining the flow rate in said cell culture system on the basis of the motion vector.

The method for determining the flow rate in the three dimensional cell culture system (2) is preferably carried out contact-free and non-invasive, particularly to avoid the direct intervention with the environment of the three dimensional cell culture system (2), e.g. the sterile, self-contained, and/or circulation three dimensional cell culture system (2). The contact-free and non-invasive determination of the flow rate is particularly conducted using optical means.

The use of erythrocytes as detectors for the determination of the flow rate in the three dimensional cell culture system (2) has the advantage that no mechanical measurement is required. That means that no foreign matter has to be introduced in the three dimensional cell culture system (2) which may contaminate or disturb the sensible and/or sterile atmosphere in said system. In addition, the dimensions of the self-contained and/or circulation three dimensional cell culture system (2) support the continuous circulation of low amounts of fluid, e.g. blood or medium, preferably of 4 to 15 µl and more preferably of 4 to 8 µl of fluid, e.g. blood or medium, through said system so that the use of common mechanical measuring instruments is complicated. Erythrocytes are, however, as part of the blood, already comprised in preferred embodiments of the self-contained and/or circulation three dimensional cell culture system (2) and can be detected with optical means. In addition, erythrocytes, as naturally components of organ systems, can also easily be added to the medium which perfuses the three dimensional cell culture systems (2) without destroying the sensible environment of said systems. The cells, multilayer cell assembly/assemblies, cell aggregate(s), tissue(s), organoid(s), and/or organ(s) comprised in the three dimensional cell culture system (2) are provided, in preferred embodiments, with all necessary vital components via the blood which comprises, by nature, erythrocytes. Blood is a strong buffer system. It provides all necessary proteins of the plasma to the cells, multilayer cell assembly/assemblies, cell aggregate(s), tissue(s), organoid(s), and/or organ(s), supports oxygen transport through the erythrocytes, and provides immunological activities against contaminating microorganisms through white blood cells. In addition, the use of erythrocytes in the three dimensional cell culture system (2) has the advantage that no foreign substances, e.g. chemicals, which could be toxic for the cells or which could have side effect, have to be introduced in the three dimensional cell culture system (2) to determine the flow rate in said system. Moreover, erythrocytes are red and, thus, clearly visible e.g. using a microscope such as a fluorescence microscope.

A "perfusable" three dimensional cell culture system (2) is a three dimensional cell culture system which can be perfused by a fluid such as blood or medium. It should be clear for the skilled person that said system is perfused by a fluid such as blood or medium at least at the time point at which the determination of the flow rate is made.

The perfusable three dimensional cell culture system (2) consists in its simplest form of at least one growth section (3) with a micro inlet through which a fluid containing erythrocytes, e.g. blood and/or medium, can flow in, e.g. a medium and/or blood inlet, and with a micro outlet through which a fluid containing erythrocytes can flow out, e.g. medium and/or blood outlet. Such a perfusable three dimensional cell culture system (2) may have the form of a cell culture tube or cell culture well. It is, however, preferred that the perfusable three dimensional cell culture system (2) is a system which comprises at least one growth section (3) with a micro inlet through which a fluid containing erythrocytes, e.g. blood and/or medium, can flow in, e.g. a medium and/or blood inlet, and with a micro outlet through which a fluid containing erythrocytes can flow out, e.g. medium and/or blood outlet. Such a perfusable three dimensional cell culture system (2) preferably further comprises a microfluidic channel which is connected with the inlet of the growth section (3) and a microfluidic channel which is connected with the outlet of the growth section (3). It is particularly preferred that the perfusable three dimensional cell culture system (2) is a self-contained and/or circulation three dimensional cell culture system (2) as described above.

It is further preferred that the "perfusable" three dimensional cell culture system (2), particularly the body of the "perfusable" three dimensional cell culture system (2), is made of a translucent material, e.g. glass such as calcium carbonate/sodium bicarbonate glass or quartz glass, or plastic. This allows the above described contact-free and non-invasive observation of the three dimensional cell culture system (2) using optical means. Particularly, it allows the analysis of the three dimensional cell culture system (2) from the outside.

The flow rate may be determined in different regions of the dimensional cell culture system (2) or in the three dimensional cell culture system (2), e.g. in the microfluidic channel, in the growth section (3), particularly in the growth cavity comprised in the growth section (3), in the micro inlet of the growth section (3), in the micro outlet of the growth section (3), in the microfluidic inlet channel of the growth section (3), or in the microfluidic outlet channel of the growth section (3). When determining the flow rate, a first picture of a region may be taken at a first time point and a second picture of the same region may be taken at a second time point. Alternatively, a first picture of a region may be taken at a first time point, a second picture of the same region may be taken at a second time point and a third picture of the same region may be taken at a third time point, or a first picture of a region may be taken at a first time point, a second picture of the same region may be taken at a second time point and a third picture of the same region may be taken at a third time point and a fourth picture of the same region may be taken of a fourth time point. It is preferred that when determining the flow rate, a first picture of a region is taken at a first time point and a second picture of the same region is taken at a second time point. The interval between both time points preferably amounts to 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2.0 seconds. The pictures may be taken with optical means. Preferably, the pictures are taken with a microscope, more preferably with a fluorescence microscope. It is also possible to generate the two pictures of the same regions at different time points from a videotape. The assignment of a motion vector to the at least one erythrocyte representing the positional change of said at least one erythrocyte on the at least two pictures, and the determination of the flow rate in said cell culture system on the basis of the motion vector is described in Adrian, R.J.; Westerweel, J. (2011). Particle Image Velocimetry. Adrian, R.J.; Westerweel, J. (2011). Particle Image Velocimetry. Cambridge University Press. ISBN 978-0-521-44008-0. Preferably, the pictures are analysed with the URAPIV open source PIV software. If the flow rate of more than one erythrocyte is determined in the three dimensional cell culture system (2), the mean flow rate in the three dimensional cell culture system (2) is calculated on the basis of all measurement data. Preferably, blood, particularly whole blood or a blood fraction, such as plasma or erythrocytes, or medium containing erythrocytes perfuses the three dimensional cell culture system (2).

In another preferred embodiment, the method for determining the physiological osmolality in the three dimensional cell culture system (2) using erythrocytes as detectors comprises the steps of:
(i) providing the three dimensional cell culture system (2) comprising at least one growth section (3), and
(ii) determining the shape of at least one erythrocyte, e.g. 2, 3, 4, 5, 6, 7, 8, 9, 10, 50, 100 erythrocytes, in said cell culture system at a time point during culturing of said cell culture system.

The method for determining the physiological osmolality in the three dimensional cell culture system (2) is preferably carried out contact-free and non-invasive, particularly to avoid the direct intervention with the environment of the three dimensional cell culture system (2), e.g. the sterile, self-contained, and/or circulation three dimensional cell culture system (2). The contact-free and non-invasive determination of the physiological osmolality is particularly conducted using optical means.

The term "physiological osmolality", as used herein, refers to the typical average osmotic pressure caused by solute particles, e.g. salt and/or sugar particles, which is present in a normal physiological environment, e.g. in blood. Such a physiological osmolality should also be present in the three dimensional cell culture system (2), particularly in the fluid, e.g. medium or blood, which is comprised in the three dimensional cell culture system (2). Such an environment is especially desired to allow the ideal growth, differentiation and/or maintenance of cell aggregates, tissues, organoids and/or organs in the three dimensional cell culture system (2).

The use of erythrocytes as detectors for the determination of the physiological osmolality in the three dimensional cell culture system (2) has the advantage that no mechanical measurement is required. For example, osmolality can be measured on an analytical instrument called an osmometer. That means that no foreign matter has to be introduced in the three dimensional cell culture system (2) which may contaminate or disturb the sensible and/or sterile atmosphere in said system. In addition, the dimensions of the self-contained and/or circulation three dimensional cell culture system (2) support the continuous circulation of low amounts of fluid, e.g. blood or medium, preferably of between 5 and 5000 µl and more preferably of between 20 and 200 µl of fluid, e.g. blood or medium, through said system so that the use of common mechanical measuring instruments is complicated. Erythrocytes are, however, as part of the blood, already comprised in preferred embodiments of the self-contained and/or circulation three dimensional cell culture system (2) and can be detected with optical means. In addition, erythrocytes, as naturally components of organ systems, can also easily be added to the medium which is comprised in the three dimensional cell culture systems (2) without destroying the sensible environment of said systems.

The cells, multilayer cell assembly/assemblies, cell aggregate(s), tissue(s), organoid(s), and/or organ(s) comprised in the three dimensional cell culture system (2) are provided, in preferred embodiments, with all necessary vital components via the blood which comprises, by nature, erythrocytes. In addition, the use of erythrocytes in the three dimensional cell culture system (2) has the advantage that no foreign substances, e.g. chemicals, which could be toxic for the cells or which could have side effect, have to be introduced in the three dimensional cell culture system (2) to determine the physiological osmolality in said system. Further, erythrocytes are indicators for the blood, particularly plasma, osmolality by nature (see below). Furthermore, erythrocytes are red and, thus, clearly visible e.g. using a microscope such as a fluorescence microscope.

The three dimensional cell culture system (2) may be a three dimensional cell culture system as described above. Preferably, the three dimensional cell culture system (2) may be a perfusable three dimensional cell culture system as described above. It is particularly preferred that the perfusable three dimensional cell culture system (2) is a self-contained and/or circulation three dimensional cell culture system (2) as described above. It is more particularly preferred that the three dimensional cell culture system (2), particularly the body of the three dimensional cell culture system (2), is made of a translucent material, e.g. glass, such as calcium carbonate/sodium bicarbonate glass or quartz glass, or plastic. This allows the above described contact-free and non-invasive observation of the three dimensional cell culture system (2) using optical means. Particularly, it allows the analysis of the three dimensional cell culture system (2) from the outside.

It is particularly preferred that blood, particularly whole blood or a blood fraction, such as plasma or erythrocytes, or medium containing erythrocytes perfuses the three dimensional cell culture system (2).

As mentioned above, erythrocytes are indicators for the blood, particularly plasma, osmolality by nature. The osmolality of medium comprising erythrocytes or the osmolality of blood, particularly plasma, influences the volume and shape of erythrocytes. Erythrocytes, particularly mammalian erythrocytes, are typically shaped as biconcave disks: flattened and depressed in the center, with a dumbbell-shaped cross section, and a torus-shaped rim on the edge of the disk. This distinctive biconcave shape optimizes the flow properties of blood in the large vessels, such as maximization of laminar flow and minimization of platelet scatter, which suppresses their atherogenic activity in those large vessels. The maintenance of erythrocyte shape is usually dependent on factors within the cell as well as in the external environment. If these are altered, the cell may become spherical. At least three sets of environmental circumstances have been described that result in the spherical shape: osmotic (hypotonic) swelling, discocyte-echinocyte transformation, and discocyte-stomatocyte transformation. Up to a point, all three types of shape change are reversible. Osmotic swelling occurs when erythrocytes are suspended in hypotonic solutions. Under such circumstances, the cell acquires water and swells, first becoming cup-shaped and then spherical. These changes are associated with an increase in volume while the cell surface area remains the same or increases only slightly. As the spherical shape is approached, the cell diameter decreases, an observation that demonstrates the elastic nature of the membrane. Discocyte-echinocyte transformation takes place when intracellular adenosine triphosphate (ATP) is depleted, when intracellular calcium is increased, when the cell is exposed to stored plasma, high pH, anionic detergents, lysolecithin or fatty acids. Discocyte-stomatocyte transformation occurs when red cells are exposed to low pH cationic detergents. As the change proceeds, the cell loses the indentation on one side, and the opposite dimple increases in depth, producing a cup-shaped cell. Since on fixed smears such cells appear to have a mouthlike "stoma" instead of a round area of central pallor, they are known as stomatocytes. The cell may also take a crenate form. Osmotic (hypertonic) shrinkage may result in a crenate form.

Based on the above, the erythrocytes are suitable indicators of the physiological osmolality in the three dimensional cell culture system (2), particularly of the physiological osmolality of the fluid, e.g. medium or blood, comprised in the three dimensional cell culture system (2), e.g. perfusing through or circulating in the three dimensional cell culture system (2). Especially as even only a 10% change in osmolality has a drastic influence on the optical parameters, which appears to be of the same order as for 10% hematocrit and oxygen saturation changes.

Thus, preferably, a spherical shape of the at least one erythrocyte indicates that the physiological osmolality is decreased (hypotonic condition, too low concentration of solute particles, e.g. salt and/or sugar particles), a crenate shape of the at least one erythrocyte indicates that the physiological osmolality is increased (hypertonic condition, too high concentration of solute particles, e.g. salt and/or sugar particles), or a biconcave shape of the at least one erythrocyte indicates that the physiological osmolality is stable (isotonic condition).

When the physiological osmolality is decreased, a hypotonic condition is present in the three dimensional cell culture system (2), particularly in the fluid, e.g. medium or blood, comprised in the three dimensional cell culture system (2). In other words, the concentration of solute particles, e.g. salt and/or sugar particles, is too low. Further, when the physiological osmolality is increased, a hypertonic condition is present in the three dimensional cell culture system (2), particularly in the fluid, e.g. medium or blood, comprised in the three dimensional cell culture system (2). In other words, the concentration of solute particles, e.g. salt and/or sugar particles, is too high. Furthermore, when the physiological osmolality is stable, an isotonic condition is present in the three dimensional cell culture system (2), particularly in the fluid, e.g. medium or blood, comprised in the three dimensional cell culture system (2). In other words, the salt concentration is normal/ideal.

The shape of at least one erythrocyte in said cell culture system may be determined at any time point during culturing of said cell culture system. The term "culture" refers to a complex process which allows the growth, the differentiation and/or the maintenance of cells under controlled conditions, generally outside of their natural environment in the three dimensional cell culture system (2). Preferably, the shape of at least one erythrocyte in said cell culture system is determined at periodic time points during culture, e.g. hourly or daily. It is particularly preferred that the shape of the at least one erythrocyte is determined after medium/blood change, after the addition of supplements, and/or after the addition of test compounds (see below).

The shape of the at least one erythrocyte may be analysed with optical means. Preferably, the shape of the at least one erythrocyte is analysed with a microscope, more preferably with a fluorescence microscope. Therefore, a detection software is preferably used. If the shape of more than one erythrocyte is determined in the three dimensional cell culture system (2), the mean shape of erythrocytes in the three dimensional cell culture system (2) may be calculated, e.g. on the basis of all measurement data using a suitable computer program.

In a further preferred embodiment, the method for determining the oxygen consumption in the three dimensional cell culture system (2) using erythrocytes as detectors comprises the steps of:
(i) providing the three dimensional cell culture system (2) comprising at least one growth section (3),
(ii) determining an average haemoglobin saturation of erythrocytes at a first position and at a second position in said cell culture system, and
(iii) calculating the oxygen consumption in said cell culture system on the basis of the average haemoglobin saturation at the different positions.

The method for determining the oxygen consumption in the three dimensional cell culture system (2) is preferably carried out contact-free and non-invasive, particularly to avoid the direct intervention with the environment of the three dimensional cell culture system (2), e.g. the sterile, self-contained, and/or circulation three dimensional cell culture system (2). The contact-free and non-invasive determination of the oxygen consumption is particularly conducted using optical means.

The three dimensional cell culture system (2) may be a three dimensional cell culture system as described above. Preferably, the three dimensional cell culture system (2) may be a perfusable three dimensional cell culture system as described above. It is particularly preferred that the perfusable three dimensional cell culture system (2) is a self-contained and/or circulation three dimensional cell culture system (2) as described above. It is more particularly preferred that the three dimensional cell culture system (2), particularly the body of the three dimensional cell culture system (2), is made of a translucent material, e.g. glass, such as calcium carbonate/sodium bicarbonate glass or quartz glass, or plastic. This allows the above described contact-free and non-invasive observation of the three dimensional cell culture system (2) using optical means. Particularly, it allows the analysis of the three dimensional cell culture system (2) from the outside.

It is particularly preferred that blood, particularly whole blood or a blood fraction, such as plasma or erythrocytes, or medium containing erythrocytes perfuses the three dimensional cell culture system (2).

For ideal growth, differentiation, and/or maintenance of the cell aggregate(s), tissue(s), organoid(s) and/or organ(s) comprised in the three dimensional cell culture system (2), particularly comprised in the growth section (3), gaseous medium, e.g. O₂/CO₂, has to be provided. The gaseous medium, e.g. O₂/CO₂, may be provided in an active or passive manner. For example, the gaseous medium may be supplemented via an external gaseous, e.g. O₂/CO₂, source to the three dimensional cell cultures system (2), particularly to the at least growth section (3) comprised therein. However, it is preferred that gaseous medium, e.g. O₂/CO₂, is provided to the three dimensional cell cultures system (2), particularly to the at least one growth section (3) comprised therein, in a passive manner, e.g. by diffusion into the growth section (3) through a gas permeable membrane or polymer foil and/or through the microfluidic channels from the environment. This membrane or polymer foil is preferably fluid-tight. The gas, e.g. O₂/CO₂, accumulates directly in the cell aggregate(s), tissue(s), organoid(s) and/or organ(s) comprised in the three dimensional cell culture system (2), particularly comprised in the growth section (3), and/or is dissolved in the fluid, e.g. blood or medium, comprised in the three dimensional cell culture system (2), particularly comprised in the microfluidic channels of said system. To provide optimal culture conditions, the observation of the oxygen consumption in the three dimensional cell culture system (2) is highly desirable. A high oxygen consumption indicates, for example, an aerobic energy supply and a low oxygen consumption indicates, for example, an energy supply through glycolysis.

As mentioned above, on the basis of the average haemoglobin saturation at different positions in the three dimensional cell culture system (2), the oxygen consumption in said cell culture system can be calculated.

Haemoglobin is an iron-containing oxygen-transport metalloprotein in the erythrocytes of almost all vertebrates. Haemoglobin in the blood carries oxygen from the respiratory organs (lungs or gills) to the rest of the body (i.e. the tissues) where it releases the oxygen to burn nutrients to provide energy to power the functions of the organism, and collects the resultant carbon dioxide to bring it back to the respiratory organs to be dispensed from the organism. Haemoglobin can be saturated with oxygen molecules (oxyhemoglobin), or desaturated with oxygen molecules (deoxyhemoglobin).

Generally, the average haemoglobin saturation of erythrocytes may be determined at any position in the three dimensional cell culture system (2), e.g. within the microfluidic channels. However, it is preferred that the average haemoglobin saturation of erythrocytes is determined at a position upstream of the at least one growth section (3) and at a position downstream of the at least one growth section (3). Particularly, to determine the oxygen consumption of the cell aggregate(s), tissue(s), organoid(s), or organ(s) comprised in the growth section (3) of the three dimensional cell culture system (2), it is preferred that the average haemoglobin saturation of erythrocytes is determined at a position upstream of the at least one growth section (3) and at a position downstream of the at least one growth section (3). In other words, the average haemoglobin saturation of erythrocytes is preferably measured at a position before the (oxygen-enriched) fluid, e.g. blood or medium containing erythrocytes, flows in the at least one growth section (3), e.g. at the micro-inlet or microfluidic inlet channel of the at least one growth section (3), and at a position after the (oxygen-reduced) fluid, e.g. blood or medium containing erythrocytes, flows out of the at least one growth section (3), e.g. at the micro-outlet or microfluidic outlet channel of the at least one growth section.

The haemoglobin saturation may be measured using optical means. The haemoglobin saturation is preferably measured using an infrared light source and red light source, e.g. using a light emitter with a red light and an infrared light source such as a light emitter with red light and infrared light LEDs, and a photodetector. In preferred embodiments, the three dimensional cell culture system (2) is made of a translucent material so that the light emitter with red light and infrared light source, e.g. LEDs, shines through the measuring site. As mentioned above, the measuring site is at a first and at a second position within the three dimensional cell culture system (2), preferably at a position upstream of the at least one growth section (3) and at a position downstream of the at least one growth section (3). Light is preferably sent through the measuring site via the transmission and reflectance method. In the transmission method, the light emitter and the photodetector may be opposite of each other with the measuring site in between. The light can then pass trough the site. In the reflectance method, the light emitter and the photodetector may also be next to each other on top or below, preferably below, the measuring site. The light bounces from the emitter to the detector across the site.

The haemoglobin saturation measurement is further based on the red and infrared light absorption characteristics of oxygenated and deoxygenated haemoglobin. Particularly, oxygenated haemoglobin absorbs more infrared light and allows more red light to pass through. Deoxygenated (or reduced) haemoglobin absorbs more red light and allows more infrared light to pass through. To determine the haemoglobin saturation at the first and second position in the three dimensional cell culture system (2), e.g. at the position upstream of the at least one growth section (3) and at the position downstream of the at least one growth section (3), said positions are preferably irradiated with infrared light and red light. The red light is preferably emitted at a wavelength of 600-750 nm and/or the absorption of infrared light is preferably emitted at a wavelength of 850-1000 nm. This is particularly based on the fact that red light is absorbed in the 600-750 nm wavelength band and infrared light is absorbed in the 850-1000 nm wavelength band. After the transmitted or reflected red (R) and infrared (IR) signals are passed through the first and second position in the three dimensional cell culture system (2), e.g. at the position upstream of the at least one growth section (3) and at the position downstream of the at least one growth section (3), and are received at the photodetector, the R/IR ratio is preferably calculated. The R/IR ratio determined at the first position and at the second position in the three dimensional cell culture system (2), e.g. at the position upstream of the at least one growth section (3) and at the position downstream of the at least one growth section (3), is than preferably compared to a "look-up" table or calibration curve (made up of empirical formulas) that convert the ratio to a haemoglobin saturation value. The determination of the haemoglobin saturation subsequently allows the calculation of oxygen consumption.

More preferably, the haemoglobin saturation is measured with the optical senor device (1) disclosed herein. Said optical sensor device (1) preferably comprises at least one light excitation fibre (4) and at least one light emission fibre (5), particularly an infrared emission fibre (9). As to the preferred embodiments of said optical sensor device (1), it is referred to the present disclosure. Again, the determination of the haemoglobin saturation subsequently allows the calculation of oxygen consumption.

Further, in a preferred embodiment, the method for determining and/or monitoring the vitality of the three dimensional cell culture system (2) by measuring living cell fluorescent dyes comprises the steps of:
(i) providing the three dimensional cell culture system (2) comprising at least one growth section (3) which is loaded with living cell fluorescent dyes, and
(iia) measuring the average fluorescence intensity in at least a part of the at least one growth section (3) of said cell culture system at a first time point and at a second time point during culturing of said cell culture system, and comparing the average fluorescence intensity at the second time point with the average fluorescence intensity at the first time point, and/or
(iib) measuring the average fluorescence intensity in at least a part of the at least one growth section (3) of said cell culture system at a time point during culturing of said cell culture system, and comparing the average fluorescence intensity at said time point with the average fluorescence intensity of at least one control cell culture system.

The method for determining and/or monitoring the vitality of the three dimensional cell culture system (2) is preferably carried out contact-free and non-invasive, particularly to avoid the direct intervention with the environment of the three dimensional cell culture system (2), e.g. the sterile, self-contained, and/or circulation three dimensional cell culture system (2). The contact-free and non-invasive determination and/or monitoring of the vitality is particularly conducted using optical means.

The three dimensional cell culture system (2) may be a three dimensional cell culture system as described above. Preferably, the three dimensional cell culture system (2) may be a perfusable three dimensional cell culture system as described above. It is particularly preferred that the perfusable three dimensional cell culture system (2) is a self-contained and/or circulation three dimensional cell culture system (2) as described above. It is particularly more preferred that the three dimensional cell culture system (2), particularly the body of the three dimensional cell culture system (2), is made of a translucent material, e.g. glass such as calcium carbonate/sodium bicarbonate glass or quartz glass, or plastic. This allows the above described contact-free and non-invasive observation of the three dimensional cell culture system (2) using optical means. Particularly, it allows the analysis of the three dimensional cell culture system (2) from the outside.

It is particularly preferred that blood, particularly whole blood or a blood fraction, such as plasma or erythrocytes, or medium containing erythrocytes perfuses the three dimensional cell culture system (2).

As mentioned above, the average fluorescence intensity is determined in at least a part of the at least one growth section (3), preferably in the whole at least one growth section (3), of said cell culture system. Said part of the at least one growth section (3) has preferably a size which is representative for the vitality status of the whole at least one growth section (3), and, thus, for the vitality status of the three dimensional cell culture system (2), particularly of the cell aggregate(s), tissue(s), organoid(s), and/or organ(s) comprised therein. Said part of the at least one growth section (3) may be a layer of cells, a multiple layer of cells (e.g. an area of 2, 3, 4, 5, 6, 7, 8, 9, 10, or more cell layers), or an area of 50x50x50 µm, 100x100x100 µm, 150x150x150 µm, 200x200x200 µm, 250x250x250 µm, 300x300x300 µm, 350x350x350 µm, 400x400x400 µm, 450x450x450 µm, or 500x500x500 µm.

The term "living fluorescent dyes", as used herein, refers to fluorescent molecules that are retained in living and vital cells through several generations, e.g. QTracker living fluorescent dyes are inherited by daughter cells for at least six generations. The living fluorescent dyes are inherited by daughter cells after cell fusion and are not transferred to adjacent cells in population. Said living fluorescent dyes are only retained in living cells having intact membranes. Dying cells or death cells do not have an intact cell membrane and, thus, are not able to incorporate the living fluorescent dyes. The cell membrane of dying or death cells is, for example, leaky or permeable due to wholes and cracks therein. Said cells are not vital anymore or have a reduced vitality. Generally, living fluorescent dyes are rapidly lost under all conditions that cause cell lysis, e.g. cell apoptosis. Thus, the fluorescence intensity of living fluorescence dyes measured at different time points during culture represents a significant indicator of vitality of the three dimensional cell culture system (2), particularly of the cell aggregate(s), tissue(s), organoids(s) and/or organ(s) comprised therein (see below).

The living fluorescent dyes can be loaded into the three dimensional cell culture system (2) by adding the reagent to the fluid, e.g. blood or medium, comprised therein. For example, the living fluorescent dyes can be loaded into the three dimensional cell culture system (2) by adding the reagent to fluid reservoir such as blood or medium reservoir. It is also preferred that the three dimensional cell culture system (2), e.g. self-contained and/or circulation three dimensional cell culture system (2), is loaded with the fluorescent dyes via an injection port. As mentioned above, the three dimensional cell culture system (2), e.g. self-contained and/or circulation three dimensional cell culture system (2), may comprise an injection port through which substances, e.g. test compounds, nutrients, fluids, chemicals such as living fluorescent dyes, can be discontinuously applied during the course of incubation. Said injection port is preferably located in a microfluidic transport channel or connected to the at least one growth section (3).

The living fluorescent dyes may pass freely through cell membranes but once inside the cells, they are transformed into cell-impermeant reaction products. CellTracker living fluorescent dyes are particularly preferred. The CellTracker living fluorescent dyes, for example, contain a chloromethyl or bromomethyl group that reacts with thiols, e.g. in a glutathione S-transferase-mediated reaction. In most of the cells, glutathione levels are high (up to 10 mM) and glutathione S-transferase is ubiquitous. Said dyes are preferably transformed into cell-impermeant fluorescent dye-thioether adducts which fluorescence can be detected. CellTracker living fluorescent dyes include the blue-fluorescent chloromethyl derivatives of amino-, hydroxy- and difluorohydroxycoumarin (CMAC, CMHC and CMF2HC), the greenfluorescent chloromethyl derivatives of fluorescein diacetate (CMFDA) and a BODIPY ® dye, the orange-fluorescent CMTMR and CMRA and the red-fluorescent CMTPX. CellTracker™ Blue CMAC, CMHC and CMF2HC, CellTracker™ Violet, the violet-fluorescent bromomethyl derivative of coumarin (BMQC), CellTracker™ Green BODIPY, CellTracker™ Orange CMTMR, and CellTracker™ Red CMTPX do not require enzymatic cleavage to activate their fluorescence, whereas the green CMFDA and orange CMRA do require enzymatic cleavage.

Another preferred living fluorescent dye is calcein AM. The acetoxymethyl (AM) ester derivatives of fluorescent indicators and chelators make up another useful group of compounds for the study of the vitality of cells. Modification of carboxylic acids with AM ester groups results in an uncharged molecule that can permeate cell membranes. Once inside the cell, the lipophilic blocking groups are cleaved by nonspecific esterases, resulting in a charged form that leaks out of cells far more slowly than its parent compound. Calcein AM is retained in cells that have intact membranes. It does not label dead cells, and is rapidly lost under conditions that cause cell lysis. This property allows to conduct flow cytometric or fluorescence-based assays for cell vitality.

Qtracker living fluorescent dyes are also preferred. Once inside the cells, Qtracker living fluorescent dyes provide intense, stable fluorescence that can be traced through several generations, and are not transferred to adjacent cells in a population.

CellMask molecules or FluoSpheres may also be used to stain living cells.

It is preferred that the method for monitoring the vitality of the three dimensional cell culture system (2) by measuring living cell fluorescent dyes comprises the steps of:
(i) providing the three dimensional cell culture system (2) comprising at least one growth section (3) which is loaded with living cell fluorescent dyes, and
(ii) measuring the average fluorescence intensity (of said living cell fluorescent dyes) in at least a part of the at least one growth section (3) of said cell culture system at a first time point and at a second time point during culturing of said cell culture system, and comparing the average fluorescence intensity at the second time point with the average fluorescence intensity at the first time point.

The interval between both time points, i.e. between the first and the second time point during culture, preferably amounts 1, 2, 3, 4, 5, 6, 7, 8, 9, 10, 11, 12, 13, 14, 15, 16, 17, 18, 19, 20, 21, 22, 23 hour(s), 1, 2, 3, 4, 5, 6 day(s), 1, 2, 3 week(s), 1, 2, 3, 4, 5, 6, 7, 8, 9, 10, 11 month(s), or 1 year. The term "culture" refers to a complex process which allows the growth, the differentiation and/or the maintenance of cells under controlled conditions, generally outside of their natural environment in the three dimensional cell culture system (2).

It is particularly preferred that a decrease of the average fluorescence intensity at the second time point when compared to the average fluorescence intensity at the first time point indicates that the vitality of said cell culture system (2) is decreased.

It is also particularly preferred that a retention of the average fluorescence intensity at the second time point when compared to the average fluorescence intensity at the first time point indicates that the vitality of said cell culture system (2) is maintained.

The three dimensional cell culture system (2), particularly the cell aggregate(s), tissue(s), organoid(s) and/or organ(s) comprised therein, may be further exposed to a test compound, particularly to one or more test compound(s) or a test compound composition, e.g. in order to conduct test compound long-term exposition studies. This allows, for example, to determine whether said test compound(s) or test compound composition has (have) an impact on the vitality of the three dimensional cell culture system (2), particularly on the vitality of the cell aggregate(s), tissue(s), organoid(s) and/or organ(s) comprised therein, e.g. whether the test compound(s) or the test compound composition has (have) side-effects, e.g. inhibit(s) the growth and/or differentiation of the cells, or is (are) toxic to the cells.

The application of a test compound to the microfluidic circuit, for example, may resemble the intravenous administration of the test compound, the application of a test compound to skin comprised in the growth section (3), may resemble the dermal administration of the test compound, the application of a test compound to the lung, represents the administration of the test compound by inhalation, and the application of a test compound to the intestine, may represent the oral administration of the test compound.

Thus, it is further/additionally preferred that said cell culture system (2) is treated with a test compound, particularly with one or more test compound(s) or with a test compound composition.

The term "test compound", as used herein, means any compound suitable for pharmaceutical delivery. The test compound is preferably selected from the group consisting of cells, viruses, bacteria, genetically modified cells, nucleic acids (e.g. vectors comprising a transgene), proteins, peptides, hormones, antibodies, RNA, preferably siRNA or dsRNA, small molecules such as small organic or inorganic molecules, preferably about 800 Daltons more preferably about 500 Daltons, drugs, pharmaceutically active substances, metabolites, natural compounds, or samples of soil, plants or marine origin. Test compounds may be designed specifically or may be derived from libraries already available. The term "library", as used herein, refers to a collection of samples. Preferably, the test compound is provided in form of a chemical compound library. Chemical compound libraries include a plurality of chemical compounds and have been assembled from any of multiple sources, including chemical synthesized molecules or natural products, or have been generated by combinatorial chemistry techniques. They are especially suitable for high-throughput screening and may be comprised of chemical compounds of a particular structure or compounds of a particular organism such as a plant. For instance, synthetic compound libraries are commercially available from Maybridge Chemical Co. (Trevillet, Cornwall, UK), ChemBridge Corporation (San Diego, CA), or Aldrich (Milwaukee, WI). A natural compound library is, for example, available from TimTec LLC (Newark, DE). Alternatively, libraries of natural compounds in the form of bacterial, fungal, plant, and animal extracts can be used. The test compound is preferably selected from the group consisting of a dermatic agent, a cardiovascular agent, a hepatic agent, an intestine agent and a neurotoxic agent. The dermatic agent is more preferably selected from the group consisting of cosmetic ingredients and consumer product chemicals, the cardiovascular agent is more preferably selected from the group consisting of heart muscle contractors and heart muscle relaxators, the hepatic agent is more preferably selected from the group consisting of metabolic phase I modulating agents or metabolic phase II modulating agents, the intestine agent is more preferably selected from the group consisting of food additives and the neurotoxic agent is more preferably selected from the group consisting of peripheral nervous system neurotoxicants and central nervous system neurotoxicants.

The test compound, particularly the one or more test compound(s) or the test compound composition, can be loaded into the three dimensional cell culture system (2) by adding the test compound, particularly the one or more test compound(s) or the test composition, to the fluid reservoir such as blood or medium reservoir. It is also preferred that the three dimensional cell culture system (2), e.g. self-contained and/or circulation three dimensional cell culture system (2), is loaded with the test compound, particularly with the one or more test compound(s) or with the test compound composition, via an injection port. As mentioned above, the three dimensional cell culture system (2), e.g. self-contained and/or circulation three dimensional cell culture system (2), may comprise an injection port through which substances, e.g. test compounds, nutrients, fluids, chemicals such as living fluorescent dyes, can be discontinuously applied during the course of incubation. Said injection port is preferably located in a microfluidic transport channel or connected to the at least one growth section (3).

It is particularly preferred that the test compound, particularly the one or more test compound(s) or the test compound composition, is (are) loaded into the three dimensional cell culture system (2) before the average fluorescence intensity is determined at the second time point, e.g. 8, 7, 6, 5, 4, 3, 2, 1 month(s), 3, 2, 1 week(s), 6, 5, 4, 3, 2, 1 day(s), 24, 23, 22, 21, 20, 19, 18, 17, 16, 15, 14, 13, 12, 11, 10, 9, 8, 7, 6, 5, 4, 3, 2, 1 hour(s) before or immediately before the determination of the average fluorescence intensity at the second time point. It is also particularly preferred that the test compound, particularly the one or more test compound(s) or the test compound composition, is (are) loaded into the three dimensional cell culture system (2) after the average fluorescence intensity is determined at the first time point, e.g. 8, 7, 6, 5, 4, 3, 2, 1 month(s), 3, 2, 1 week(s), 6, 5, 4, 3, 2, 1 day(s), 24, 23, 22, 21, 20, 19, 18, 17, 16, 15, 14, 13, 12, 11, 10, 9, 8, 7, 6, 5, 4, 3, 2, 1 hour(s) after or immediately after the determination of the average fluorescence intensity at the first time point, preferably immediately after the determination of the average fluorescence intensity at the first time point. In this way, the effect of the test compound, particularly the one or more test compound(s) on the three dimensional cell culture system can be studied.

The test compound may have an influence or may have no influence on the average fluorescence intensity of the living fluorescent dyes. An alteration or a retention of the average fluorescence intensity of the living fluorescent dyes may be indicative for the vitality of said cell culture system (2).

It is particularly preferred that a decrease of the average fluorescence intensity at the second time point when compared to the average fluorescence intensity at the first time point indicates that the vitality of said cell culture system (2) is decreased.

It is also particularly preferred that a retention of the average fluorescence intensity at the second time point when compared to the average fluorescence intensity at the first time point indicates that the vitality of said cell culture system (2) is maintained.

It is also, additionally or alternatively, preferred that the method for determining the vitality of the three dimensional cell culture system (2) by measuring living cell fluorescent dyes comprises the steps of:
(i) providing the three dimensional cell culture system (2) comprising at least one growth section (3) which is loaded with living cell fluorescent dyes, and
(ii) measuring the average fluorescence intensity (of said living cell fluorescent dyes) in at least a part of the at least one growth section (3) of said cell culture system at a time point during culturing of said cell culture system, and comparing the average fluorescence intensity at said time point with the average fluorescence intensity of at least one control cell culture system.

The average fluorescence intensity may be measured at a time point 1, 2, 3, 4, 5, 6, 7, 8, 9, 10, 11, 12, 13, 14, 15, 16, 17, 18, 19, 20, 21, 22, 23 hour(s), 1, 2, 3, 4, 5, 6 day(s), 1, 2, 3 week(s), or 1, 2, 3, 4, 5, 6, 7, 8, 9, 10, 11 month(s) or 1 year after the start of the cultivation process of the three dimensional cell culture system (2). The term "culture" refers to a complex process which allows the growth, the differentiation and/or the maintenance of cells under controlled conditions, generally outside of their natural environment in the three dimensional cell culture system (2).

The control cell culture system may be any system which allows, by comparing its average fluorescence intensity with the average fluorescence intensity of the three dimensional cell culture system (2), the formulation of a statement as to the vitality of the three dimensional cell culture system (2). The control cell culture system is preferably not loaded with living cell fluorescent dyes, preferably with the specific living cell fluorescent dyes as mentioned above. It is preferred that the control cell culture system which is not loaded with living cell fluorescent dyes is a three dimensional cell culture system as described above, e.g. a self-contained and/or circulation three dimensional cell culture system as described above. It is more preferred that the control cell culture system is the same three dimensional cell culture system as provided in step (i) but not loaded with living cell fluorescent dyes.

It is particularly preferred that an increase of the average fluorescence intensity at said time point compared to the average fluorescence intensity of the control cell culture system indicates that said cell culture system (2) is vital.

It is also particularly preferred that the comparability of the average fluorescence intensity at said time point with the average fluorescence intensity of the control cell culture system indicates that said cell culture system (2) is not vital.

As mentioned above, the three dimensional cell culture system (2), particularly the cell aggregate(s), tissue(s), organoid(s) and/or organ(s) comprised therein, may further be exposed to a test compound, particularly to one or more test compound(s) or to a test compound composition, e.g. in order to conduct test compound long-term exposition studies. This allows, for example, to determine whether said test compound(s) or test compound composition has (have) an impact on the vitality of the three dimensional cell culture system (2), particularly on the vitality of the cell aggregate(s), tissue(s), organoid(s) and/or organ(s) comprised therein, e.g. whether the test compound(s) or test compound composition has (have) side-effects, e.g. inhibit(s) the growth and/or differentiation of the cells, or is (are) toxic to the cells.

Thus, it is further/additionally preferred that said cell culture system (2) is treated with a test compound, particularly with one or more test compound(s) or with a test compound composition, and that the control cell culture system is not treated with a test compound, particularly with one or more test compound(s) or with a test compound composition.

As to the definition of the terms "test compound" and "library", the preferred embodiments of the "test compound" and as to the specific forms of "test compound" loading into the three dimensional cell culture system (2), it is referred to the above definitions and explanations.

The control cell culture system not treated with the test compound is preferably a three dimensional cell culture system. It is preferred that the control cell culture system which is not treated with the test compound is a three dimensional cell culture system as described above, e.g. a self-contained and/or circulation three dimensional cell culture system as described above. It is more preferred that the control cell culture system is the same three dimensional cell culture system as provided in step (i) but not treated with the test compound. It is mostly preferred that the above mentioned control cell culture systems are not loaded with living cell fluorescent dyes, preferably with the specific living cell fluorescent dyes as mentioned above.

The test compound may have an influence or may have no influence on the average fluorescence intensity of the living fluorescent dyes. An alteration or a retention of the average fluorescence intensity of the living fluorescent dyes may be indicative for the vitality of said cell culture system (2).

It is particularly preferred that an increase of the average fluorescence intensity at said time point compared to the average fluorescence intensity of the control cell culture system (particularly not loaded with the test compound and/or not loaded with living cell fluorescent dyes) indicates that said cell culture system (2) is vital.

It is also particularly preferred that the comparability of the average fluorescence intensity at said time point with the average fluorescence intensity of the control cell culture system (particularly not loaded with the test compound and/or not loaded with living cell fluorescent dyes) indicates that said cell culture system (2) is not vital.

It is preferred that the culturing conditions of the above described control cell culture system and three dimensional cell culture system (2) are identical and/or that the measuring times in both systems are squared with each other in order to improve the comparability of the measuring data.

Preferably, the determination/measurement of the average autofluorescence intensity is performed by spectrometry (e.g. UV/VIS spectrometry), preferably using a fluorometer, fluorescence reader, and/or fluorescence microscope. More preferably, the average autofluorescence intensity is determined/measured using the optical sensor device (1) disclosed herein. Said optical sensor device (1) preferably comprises at least one light excitation fibre (4) and at least one light emission fibre (5), particularly a fluorescence emission fibre (8). As to the preferred embodiments of said optical sensor device (1), it is referred to the present disclosure.

Furthermore, in a preferred embodiment, the method for determining and/or monitoring the metabolism status of the three dimensional cell culture system (2) by measuring the autofluorescence of NADH and/or FAD, and/or by determining the NADH/NAD⁺ and/or NADH/FAD ratio comprises the steps of:
(i) providing the three dimensional cell culture system (2) comprising at least one growth section (3),
(ii) activating NADH and/or FAD comprised in the at least one growth section (3) of said cell culture system (preferably with UV light), and
(iiia) measuring the average autofluorescence intensity of NADH, measuring the average autofluorescence intensity of FAD, determining the NADH/NAD⁺ ratio and/or determining the NADH/FAD ratio in at least a part of the at least one growth section (3) of said cell culture system at a first time point and at a second time point during culturing of said cell culture system, and
   comparing the average autofluorescence intensity of NADH, the average autofluorescence intensity of FAD, the NADH/NAD⁺ ratio and/or the NADH/FAD ratio at the second time point with the average fluorescence intensity of NADH, the average autofluorescence intensity of FAD, the NADH/NAD⁺ ratio and/or the NADH/FAD ratio at the first time point, and/or
(iiib) measuring the average autofluorescence intensity of NADH, measuring the average autofluorescence intensity of FAD, determining the NADH/NAD⁺ ratio and/or determining the NADH/FAD ratio in at least a part of the at least one growth section (3) of said cell culture system at a time point during culturing of said cell culture system, and
   comparing the average autofluorescence intensity of NADH, the average autofluorescence intensity of FAD, the NADH/NAD⁺ ratio and/or the NADH/FAD ratio at said time point with the average autofluorescence intensity of NADH, the average autofluorescence intensity of FAD, the NADH/NAD⁺ ratio and/or the NADH/FAD ratio of a control cell culture system.

The method for determining and/or monitoring the metabolism status of the three dimensional cell culture system (2) is preferably carried out contact-free and non-invasive, particularly to avoid the direct intervention with the environment of the three dimensional cell culture system (2), e.g. the sterile, self-contained, and/or circulation three dimensional cell culture system (2). The contact-free and non-invasive determination and/or monitoring of the metabolism status is particularly conducted using optical means.

The three dimensional cell culture system (2) may be a three dimensional cell culture system as described above. Preferably, the three dimensional cell culture system (2) may be a perfusable three dimensional cell culture system as described above. It is particularly preferred that the perfusable three dimensional cell culture system (2) is a self-contained and/or circulation three dimensional cell culture system (2) as described above. It is particularly more preferred that the three dimensional cell culture system (2), particularly the body of the three dimensional cell culture system (2), is made of a translucent material, e.g. glass such as calcium carbonate/sodium bicarbonate glass or quartz glass, or plastic. This allows the above described contact-free and non-invasive observation of the three dimensional cell culture system (2) using optical means. Particularly, it allows the analysis of the three dimensional cell culture system (2) from the outside.

It is particularly preferred that blood, particularly whole blood or a blood fraction, such as plasma or erythrocytes, or medium containing erythrocytes perfuses the three dimensional cell culture system (2).

As mentioned above, the average fluorescence intensity is determined in at least a part of the at least one growth section (3), preferably in the whole at least one growth section (3), of said cell culture system. Said part of the at least one growth section (3) has preferably a size which is representative for the vitality status of the whole at least one growth section (3), and, thus, for the vitality status of the three dimensional cell culture system (2), particularly of the cell aggregate(s), tissue(s), organoid(s), and/or organ(s) comprised therein. Said part of the at least one growth section (3) may be a layer of cells, a multiple layer of cells (e.g. an area of 2, 3, 4, 5, 6, 7, 8, 9, 10, or more cell layers), or an area of 50x50x50 µm, 100x100x100 µm, 150x150x150 µm, 200x200x200 µm, 250x250x250 µm, 300x300x300 µm, 350x350x350 µm, 400x400x400 µm, 450x450x450 µm, or 500x500x500 µm.

The term "cell metabolism", as used herein, means the total of all the chemical processes that occur in living cells resulting in growth, production of energy, elimination of waste material and detoxification of external substances. In the method of the present invention, the metabolism status of the three dimensional cell culture system (2), particularly of the multilayer cell assembly/assemblies, cell aggregate(s), tissue(s), organoid(s), and/or organ(s) comprised in the three dimensional cell culture system (2) is determined and/or monitored by measuring the autofluorescence of NADH and/or FAD, and/or by determining the NADH/NAD⁺ and/or NADH/FAD ratio.

The term "autofluorescence", as used herein, means the natural emission of light by biological structures such as mitochondria and lysosomes when they have absorbed light. The most commonly observed autofluorescencing molecules are NADPH (NADH = reduced form of nicotinamide adenine dinucleotide) and FAD (FAD = oxidised form of flavin adenine dinucleotide). The metabolism within a cell and, thus, in a cell aggregate, tissue, organoid or organ can be characterized due to the ratio of the NADH/NAD⁺ (NAD⁺ = oxidised form of nicotinamide adenine dinucleotide) and/or NADH/FAD ratio. Said molecules functions in the basic mechanisms of energy generation such as glycolysis, citric acid cycle and respiratory chain as electron transporter in order to permit the conversion of Adenosine-diphosphate (ADP) to Adenosine-triphosphate (ATP) during oxidative phosphorylation. The redox-pairs NADH/NAD⁺ and FAD/FADH₂ (FADH₂ = reduced form of flavin adenine dinucleotide) have, dependent on their oxidation state, a different fluorescence behaviour. In the redox-pair NADH/NAD⁺, NADH is usually fluorescent between about 400 and 490 nm, if an excitation between about 320 and 350 nm takes place. In contrast to NADH, NAD is not fluorescent. In the redox-pair FAD/FADH₂, FAD is usually fluorescent between about 500 and 560 nm, if an excitation between about 300 and 380 nm or between about 430 and 480 nm takes place. In contrast to FAD, FADH₂ is not fluorescent. As only NADH and not NAD+ has this fluorescence behaviour, it is possible to distinguish between these both redox-components and as only FAD and not FADH₂ has this fluorescence behaviour, it is possible to distinguish between these both redox-components. The ratio of NAD⁺ to NADH usually amounts to 0.05 within a cell. Due to the central position of NADH and NAD⁺ as well as FAD and FADH₂ within the function of a cell, it is already possible to make a decision about the intracellular redox potential as well as about the status of energy generation and, thus, about the cell metabolism on the basis of NADH/NAD⁺ ratio and/or NADH/FAD ratio. A high NADH concentration, for example, amounts to a reduced efficiency of the mitochondrial respiratory chain. In addition, only the NADH/NAD⁺ ratio shows a specific change depending on the mitochondrial energy generation.

The NADH/NAD⁺ ratio or the NADH/FAD ratio is preferably determined with spectrometry, e.g. with a UV light source such as a UV laser and a UV/VIS spectrometer. For this purpose, a light emitter connected to a UV/VIS spectrometer may be used which is brought in contact with the measuring site. For NADH, the cells in the area of the measuring site may be excited with a UV laser having a wavelength of 337 nm. The autofluorescence of NADH may be measured at a wavelength of 450 or 460 nm using a photomultiplier. For FAD, the cells in the area of the measuring cite may be excited with a UV laser having a wavelength of 330 nm (first absorption peak). The autofluorescence of FAD may be measured at a wavelength of 520 or 530 nm using a photomultiplier. For FAD, the cells in the area of the measuring cite may further be excited with a UV laser having a wavelength of 450 nm (second absorption peak). The autofluorescence of FAD may also be measured at a wavelength of 520 or 530 nm using a photomultiplier.

NADH is preferably activated at a wavelength of between about 320 and 350 nm, more preferably between about 320 and 340 nm, and most preferably at a wavelength of 337 nm, e.g. using a UV light source, and the autofluorescence of NADH is preferably measured at a wavelength of between about 400 and 490 nm, more preferably between about 420 and 460 nm, and most preferably at a wavelength of 450 or 460 nm, e.g. using a UV/VIS spectrometer. FAD is preferably activated at a wavelength of between about 300 and 380 nm, more preferably between about 330 and 360 nm, and most preferably at a wavelength of 330 nm, or at a wavelength of between about 430 and 480 nm, more preferably between about 440 and 460 nm, and most preferably at a wavelength of 450 nm, e.g. using a UV light source, and the autofluorescence of FAD is preferably measured at a wavelength of between about 500 and 560 nm, more preferably between about 520 and 540 nm, and most preferably at a wavelength of 520 or 530 nm, e.g. using a UV/VIS spectrometer.

It is particularly preferred that the average autofluorescence intensity of NADH and/or FAD is measured and/or that the NADH/NAD⁺ and/or NADH/FAD ratio is determined using the optical sensor device (1) disclosed herein. Said optical sensor device (1) preferably comprises at least one light excitation fibre (4) and at least one light emission fibre (5). More preferably, said optical sensor device (1) comprises (i) a UV light excitation fibre, (ia) which particularly allows the activation of NADH at a wavelength of between about 320 and 350 nm, e.g. at 337 nm, (ib) which particularly allows the activation of FAD at a wavelength of between about 300 and 380 nm, e.g. at 330 nm, and/or at a wavelength of between about 430 and 480 nm, e.g. at 450 nm, or (ic) which particularly allows the activation of NADH at a wavelength of between about 320 and 350 nm, e.g. at 337 nm, and which particularly allows the activation of FAD at a wavelength of between about 300 and 380 nm, e.g. at 330 nm, and/or at a wavelength of between about 430 and 480 nm, e.g. at 450 nm, (ii) an NADH emission fibre (6), which particularly allows the detection of the autofluorescence of NADH at a wavelength of between about 400 and 490 nm, e.g. at 450 or 460 nm, and/or (iii) a FAD emission fibre (7), which particularly allows the detection of the autofluorescence of FAD at a wavelength of between about 500 and 560 nm., e.g. at 520 or 530 nm. As to the preferred embodiments of said optical sensor device (1), it is referred to the present disclosure.

It is preferred that the method for monitoring the metabolism status of the three dimensional cell culture system (2) by measuring the autofluorescence of NADH and/or FAD, and/or by determining the NADH/NAD⁺ and/or NADH/FAD ratio comprises the steps of:
(i) providing the three dimensional cell culture system (2) comprising at least one growth section (3),
(ii) activating NADH and/or FAD comprised in the at least one growth section (3) of said cell culture system (with UV light), and
(iii) measuring the average autofluorescence intensity of NADH, measuring the average autofluorescence intensity of FAD, determining the NADH/NAD⁺ ratio and/or determining the NADH/FAD ratio in at least a part of the at least one growth section (3) of said cell culture system at a first time point and at a second time point during culturing of said cell culture system, and
   comparing the average autofluorescence intensity of NADH, the average autofluorescence intensity of FAD, the NADH/NAD⁺ ratio and/or the NADH/FAD ratio at the second time point with the average fluorescence intensity of NADH, the average autofluorescence intensity of FAD, the NADH/NAD⁺ ratio and/or the NADH/FAD ratio at the first time point.

The interval between both time points, i.e. between the first and the second time point during culture, preferably amounts 1, 2, 3, 4, 5, 6, 7, 8, 9, 10, 11, 12, 13, 14, 15, 16, 17, 18, 19, 20, 21, 22, 23 hour(s), 1, 2, 3, 4, 5, 6 day(s), 1, 2, 3 week(s), 1, 2, 3, 4, 5, 6, 7, 8, 9, 10, 11 month(s), or 1 year. The term "culture" refers to a complex process which allows the growth, the differentiation and/or the maintenance of cells under controlled conditions, generally outside of their natural environment in the three dimensional cell culture system (2).

It is particularly preferred that a retention of the average autofluorescence intensity of NADH, the average autofluorescence intensity of FAD, the NADH/NAD⁺ ratio and/or the NADH/FAD ratio at the second time point when compared to the average fluorescence intensity of NADH, the average autofluorescence intensity of FAD, the NADH/NAD⁺ ratio and/or the NADH/FAD ratio at the first time point indicates that the metabolism of said cell culture system (2) is stable.

It is further particularly preferred that
(i) a decrease of the average autofluorescence intensity of NADH at the second time point when compared to the average fluorescence intensity of NADH at the first time point indicates that the metabolism of said cell culture system (2) is increased,
(ii) a decrease of the NADH/NAD⁺ ratio at the second time point when compared to the NADH/NAD⁺ ratio at the first time point indicates that the metabolism of said cell culture system (2) is increased,
(iii) a decrease of the NADH/FAD ratio at the second time point when compared to the NADH/FAD ratio at the first time point indicates that the metabolism of said cell culture system (2) is increased, and/or
(iv) an increase of the average autofluorescence intensity of FAD at the second time point when compared to the average fluorescence intensity of FAD at the first time point indicates that the metabolism of said cell culture system (2) is increased.

It is also particularly preferred that
(i) an increase of the average autofluorescence intensity of NADH at the second time point when compared to the average fluorescence intensity of NADH at the first time point indicates that the metabolism of said cell culture system (2) is decreased,
(ii) an increase of the NADH/NAD⁺ ratio at the second time point when compared to the NADH/NAD⁺ ratio at the first time point indicates that the metabolism of said cell culture system (2) is decreased,
(iii) an increase of the NADH/FAD ratio at the second time point when compared to the NADH/FAD ratio at the first time point indicates that the metabolism of said cell culture system (2) is decreased, and/or
(iv) a decrease of the average autofluorescence intensity of FAD at the second time point when compared to the average fluorescence intensity of FAD at the first time point indicates that the metabolism of said cell culture system (2) is decreased.

The three dimensional cell culture system (2), particularly the cell aggregate(s), tissue(s), organoid(s) and/or organ(s) comprised therein, may further be exposed to a test compound, particularly to one or more test compound(s) or a test compound composition, e.g. in order to conduct test compound long-term exposition studies. This allows, for example, to determine whether said test compound(s) or test compound composition has (have) an impact on the metabolism of the three dimensional cell culture system (2), particularly an impact on the metabolism of the cell aggregate(s), tissue(s), organoid(s) and/or organ(s) comprised therein, e.g. whether the test compound(s) or the test compound composition has (have) an influence on NADH, FAD, the NADH/FAD ratio and/or the NADH/NAD⁺ ratio.

The application of a test compound to the microfluidic circuit, for example, may resemble the intravenous administration of the test compound, the application of a test compound to skin comprised in the growth section (3), may resemble the dermal administration of the test compound, the application of a test compound to the lung, represents the administration of the test compound by inhalation, and the application of the test compound to the intestine may represent the oral administration of the test compound.

Thus, it is further/additionally preferred that said cell culture system (2) is treated with a test compound, particularly with one or more test compound(s) or with a test compound composition.

As to the definition of the terms "test compound" and "library", the preferred embodiments of the "test compound" and as to the specific forms of "test compound" loading into the three dimensional cell culture system (2), it is referred to the above definitions and explanations.

It is particularly preferred that the test compound, particularly the one or more test compound(s) or the test compound composition, is (are) loaded into the three dimensional cell culture system (2) before the average autofluorescence intensity of NADH is measured, before the average autofluorescence intensity of FAD is measured, before the NADH/NAD⁺ ratio is determined and/or before the NADH/FAD ratio is determined at the second time point, e.g. 8, 7, 6, 5, 4, 3, 2, 1 month(s), 3, 2, 1 week(s), 6, 5, 4, 3, 2, 1 day(s), 24, 23, 22, 21, 20, 19, 18, 17, 16, 15, 14, 13, 12, 11, 10, 9, 8, 7, 6, 5, 4, 3, 2, 1 hour(s) before or immediately before the average autofluorescence intensity of NADH is measured, before the average autofluorescence intensity of FAD is measured, before the NADH/NAD⁺ ratio is determined and/or before the NADH/FAD ratio is determined at the second time point. It is also particularly preferred that the test compound, particularly the one or more test compound(s) or the test compound composition, is (are) loaded into the three dimensional cell culture system (2) after the average autofluorescence intensity of NADH is measured, after the average autofluorescence intensity of FAD is measured, after the NADH/NAD⁺ ratio is determined and/or after the NADH/FAD ratio is determined at the first time point, e.g. 8, 7, 6, 5, 4, 3, 2, 1 month(s), 3, 2, 1 week(s), 6, 5, 4, 3, 2, 1 day(s), 24, 23, 22, 21, 20, 19, 18, 17, 16, 15, 14, 13, 12, 11, 10, 9, 8, 7, 6, 5, 4, 3, 2, 1 hour(s) after or immediately after the average autofluorescence intensity of NADH is measured, after the average autofluorescence intensity of FAD is measured, after the NADH/NAD⁺ ratio is determined and/or after the NADH/FAD ratio is determined at the first time point, preferably immediately after the above measurement(s) and/or determination(s) at the first time point. In this way, the effect of the test compound, particularly the one or more test compound(s) on the three dimensional cell culture system can be studied.

The test compound may have an influence or may have no influence on the average autofluorescence intensity of NADH, the average autofluorescence intensity of FAD, the NADH/NAD⁺ ratio, and/or NADH/FAD ratio. An alteration of said average autofluorescence intensity/intensities and/or said ratio(s) may be indicative for the metabolism status of said cell culture system (2).

It is particularly preferred that
(i) a decrease of the average autofluorescence intensity of NADH at the second time point when compared to the average fluorescence intensity of NADH at the first time point indicates that the metabolism of said cell culture system (2) is increased,
(ii) a decrease of the NADH/NAD⁺ ratio at the second time point when compared to the NADH/NAD⁺ ratio at the first time point indicates that the metabolism of said cell culture system (2) is increased,
(iii) a decrease of the NADH/FAD ratio at the second time point when compared to the NADH/FAD ratio at the first time point indicates that the metabolism of said cell culture system (2) is increased, and/or
(iv) an increase of the average autofluorescence intensity of FAD at the second time point when compared to the average fluorescence intensity of FAD at the first time point indicates that the metabolism of said cell culture system (2) is increased.

It is also particularly preferred that
(i) an increase of the average autofluorescence intensity of NADH at the second time point when compared to the average fluorescence intensity of NADH at the first time point indicates that the metabolism of said cell culture system (2) is decreased,
(ii) an increase of the NADH/NAD⁺ ratio at the second time point when compared to the NADH/NAD⁺ ratio at the first time point indicates that the metabolism of said cell culture system (2) is decreased,
(iii) an increase of the NADH/FAD ratio at the second time point when compared to the NADH/FAD ratio at the first time point indicates that the metabolism of said cell culture system (2) is decreased, and/or
(iv) a decrease of the average autofluorescence intensity of FAD at the second time point when compared to the average fluorescence intensity of FAD at the first time point indicates that the metabolism of said cell culture system (2) is decreased.

It is also, additionally or alternatively, preferred that the method for determining the metabolism status of the three dimensional cell culture system (2) by measuring the autofluorescence of NADH and/or FAD, and/or by determining the NADH/NAD⁺ and/or NADH/FAD ratio comprises the steps of:
(i) providing the three dimensional cell culture system (2) comprising at least one growth section (3),
(ii) activating NADH and/or FAD comprised in the at least one growth section (3) of said cell culture system (with UV light), and
(iii) measuring the average autofluorescence intensity of NADH, measuring the average autofluorescence intensity of FAD, determining the NADH/NAD⁺ ratio and/or determining the NADH/FAD ratio in at least a part of the at least one growth section (3) of said cell culture system at a time point during culturing of said cell culture system, and
   comparing the average autofluorescence intensity of NADH, the average autofluorescence intensity of FAD, the NADH/NAD⁺ ratio and/or the NADH/FAD ratio at said time point with the average autofluorescence intensity of NADH, the average autofluorescence intensity of FAD, the NADH/NAD⁺ ratio and/or the NADH/FAD ratio of a control cell culture system.

The average fluorescence intensity of NADH may be measured, the average fluorescence intensity of FAD may be measured, the NADH/NAD⁺ ratio may be determined and/or the NADH/FAD ratio may be determined at a time point 1, 2, 3, 4, 5, 6, 7, 8, 9, 10, 11, 12, 13, 14, 15, 16, 17, 18, 19, 20, 21, 22, 23 hour(s), 1, 2, 3, 4, 5, 6 day(s), 1, 2, 3 week(s), or 1, 2, 3, 4, 5, 6, 7, 8, 9, 10, 11 month(s), or 1 year after the start of the cultivation process of the three dimensional cell culture system (2). The term "culture" refers to a complex process which allows the growth, the differentiation and/or the maintenance of cells under controlled conditions, generally outside of their natural environment in the three dimensional cell culture system (2).

The control cell culture system is preferably a system which represents the metabolism status at the start point of cultivation or at any other interesting point during cultivation, e.g. the point before/after fluid, e.g. blood or medium, adaptation, or the point before/after the amendment of culture conditions such as temperature, pressure, and/or pH. It is preferred that said control cell culture system is a three dimensional cell culture system as described above, e.g. a self-contained and/or circulation three dimensional cell culture system as described above. It is more preferred that the control cell culture system is the same three dimensional cell culture system as provided in step (i), e.g. representing the metabolism status at the start point of cultivation.

It is particularly preferred that the comparability of the average autofluorescence intensity of NADH, the average autofluorescence intensity of FAD, the NADH/NAD⁺ ratio and/or the NADH/FAD ratio at said time point with the average fluorescence intensity of NADH, the average autofluorescence intensity of FAD, the NADH/NAD⁺ ratio and/or the NADH/FAD ratio of the control cell culture system indicates that the metabolism of said cell culture system (2) is stable.

It is also particularly preferred that
(i) a decrease of the average autofluorescence intensity of NADH at said time point compared to the average autofluorescence intensity of NADH of the control cell culture system indicates that the metabolism of said cell culture system (2) is increased,
(ii) a decrease of the NADH/NAD⁺ ratio at said time point compared to the NADH/NAD⁺ ratio of the control cell culture system indicates that the metabolism of said cell culture system (2) is increased,
(iii) a decrease of the NADH/FAD ratio at said time point compared to the NADH/FAD ratio of the control cell culture system indicates that the metabolism of said cell culture system (2) is increased, and/or
(iv) an increase of the average autofluorescence intensity of FAD at said time point compared to the average autofluorescence intensity of FAD of the control cell culture system indicates that the metabolism of said cell culture system (2) is increased.

It is further particularly preferred that
(i) an increase of the average autofluorescence intensity of NADH at said time point compared to the average autofluorescence intensity of NADH of the control cell culture system indicates that the metabolism of said cell culture system (2) is decreased,
(ii) an increase of the NADH/NAD⁺ ratio at said time point compared to the NADH/NAD⁺ ratio of the control cell culture system indicates that the metabolism of said cell culture system (2) is decreased,
(iii) an increase of the NADH/FAD ratio at said time point compared to the NADH/FAD ratio of the control cell culture system indicates that the metabolism of said cell culture system (2) is decreased, and/or
(iv) a decrease of the average autofluorescence intensity of FAD at said time point compared to the average autofluorescence intensity of FAD of the control cell culture system indicates that the metabolism of said cell culture system (2) is decreased.

As mentioned above, the three dimensional cell culture system (2), particularly the cell aggregate(s), tissue(s), organoid(s) and/or organ(s) comprised therein, may further be exposed to a test compound, particularly to one or more test compound(s) or to a test compound composition, e.g. in order to conduct test compound long-term exposition studies. This allows, for example, to determine whether said test compound(s) or test compound composition has (have) an impact on the metabolism of the three dimensional cell culture system (2), particularly an impact on the metabolism of the cell aggregate(s), tissue(s), organoid(s) and/or organ(s) comprised therein, e.g. whether the test compound(s) or the test compound composition has (have) an influence on NADH, FAD, the NADH/FAD ratio and/or the NADH/NAD⁺ ratio.

Thus, it is further/additionally preferred that said cell culture system (2) is treated with a test compound, particularly with one or more test compound(s) or with a test compound composition, and that the control cell culture system is not treated with a test compound, particularly with one or more test compound(s) or with a test compound composition.

As to the definition of the terms "test compound" and "library", as to the preferred embodiments of the "test compound", and as to the specific forms of "test compound" loading into the three dimensional cell culture system (2), it is referred to the above definitions and explanations.

The control cell culture system not treated with the test compound is preferably a three dimensional cell culture system. It is preferred that the control cell culture system which is not treated with the test compound is a three dimensional cell culture system as described above, e.g. a self-contained and/or circulation three dimensional cell culture system as described above. It is more preferred that the control cell culture system is the same three dimensional cell culture system as provided in step (i) but not treated with the test compound.

The test compound may have an influence or may have no influence on the average autofluorescence intensity of NADH, the average autofluorescence intensity of FAD, the NADH/NAD⁺ ratio, and/or NADH/FAD ratio. An alteration or a retention of said average autofluorescence intensity/intensities and/or said ratio(s) may be indicative for the metabolism status of said cell culture system (2).

It is particularly preferred that the comparability of the average autofluorescence intensity of NADH, the average autofluorescence intensity of FAD, the NADH/NAD⁺ ratio and/or the NADH/FAD ratio at said time point with the average fluorescence intensity of NADH, the average autofluorescence intensity of FAD, the NADH/NAD⁺ ratio and/or the NADH/FAD ratio of the control cell culture system indicates that the metabolism of said cell culture system (2) is stable.

It is also particularly preferred that
(i) a decrease of the average autofluorescence intensity of NADH at said time point compared to the average autofluorescence intensity of NADH of the control cell culture system indicates that the metabolism of said cell culture system (2) is increased,
(ii) a decrease of the NADH/NAD⁺ ratio at said time point compared to the NADH/NAD⁺ ratio of the control cell culture system indicates that the metabolism of said cell culture system (2) is increased,
(iii) a decrease of the NADH/FAD ratio at said time point compared to the NADH/FAD ratio of the control cell culture system indicates that the metabolism of said cell culture system (2) is increased, and/or
(iv) an increase of the average autofluorescence intensity of FAD at said time point compared to the average autofluorescence intensity of FAD of the control cell culture system indicates that the metabolism of said cell culture system (2) is increased.

It is further particularly preferred that
(i) an increase of the average autofluorescence intensity of NADH at said time point compared to the average autofluorescence intensity of NADH of the control cell culture system indicates that the metabolism of said cell culture system (2) is decreased,
(ii) an increase of the NADH/NAD⁺ ratio at said time point compared to the NADH/NAD⁺ ratio of the control cell culture system indicates that the metabolism of said cell culture system (2) is decreased,
(iii) an increase of the NADH/FAD ratio at said time point compared to the NADH/FAD ratio of the control cell culture system indicates that the metabolism of said cell culture system (2) is decreased, and/or
(iv) a decrease of the average autofluorescence intensity of FAD at said time point compared to the average autofluorescence intensity of FAD of the control cell culture system indicates that the metabolism of said cell culture system (2) is decreased.

It is preferred that the culturing conditions of the above described control cell culture system and three dimensional cell culture system (2) are identical and/or that the measuring times in both systems are squared with each other in order to improve the comparability of the measuring data.
Preferably, the measuring of the average autofluorescence intensity is performed by spectrometry (e.g. UV/VIS spectrometry), preferably using a fluorometer, fluorescence reader, or fluorescence microscope. As already mentioned above, it is particularly preferred that the average autofluorescence intensity of NADH and/or FAD is measured and/or that the NADH/NAD⁺ and/or the NADH/FAD ratio is determined using the optical sensor device (1) disclosed herein.

The above described method can particularly be used for the *in vitro* analysis of nerve cells/nerve cell aggregates, e.g. nerve ganglia/nerve ganglia aggregates. For the establishment of an action potential in nerve cells, e.g. nerve ganglia, remarkable energy resources are required. The membrane-bound Na⁺/K⁺ ATPase establishes the excitation potential via active transport of sodium (Na⁺) in the extracellular area and potassium (K⁺) in the cytosol. The energy required for this transport is achieved via dephosphorylation of ADP. For the regeneration of the energy carrier, NADH is oxidized in the respiratory chain. Thus, with the determination of the NADH⁺/NAD ratio, as described above, it is possible to make a statement as to the excitation potential of nerve cells/nerve cell aggregates, e.g. nerve ganglia/nerve ganglia aggregates, in the three dimensional cell culture system (2), particularly in the self-contained and/or circulation three dimensional cell culture system (2) as described herein. Accordingly, with the above described method it is possible to monitor a three dimensional cell culture system (2) comprising nerve cells/nerve cell aggregates, e.g. nerve ganglia/nerve ganglia aggregates, particularly after application of a test compound.

In the above described methods, it is preferred that the at least one growth section (3) comprises a multilayer cell assembly, a cell aggregate, a tissue, an organoid, or an organ. It is more preferred that the cell aggregate is a nerve cell aggregate, particularly a nerve ganglion aggregate. It is further more preferred that the organoid is a liver lobule, nephrons of kidney, the dermis and/or epidermis of skin, gut mucosa, Langerhans islets of pancreas, grey and white matter of brain cortex and cerebellum, or adult quiescence-promoting stem cell niches. It is also more preferred that the organ is a micro-lung, micro-heart, micro-skin, micro-gut, micro-brain, micro-bone-marrow, micro-bone, micro-spleen, micro-pancreas, micro-testes, or micro-liver. It is particularly more preferred that the three dimensional cell culture system (2) comprises more than one growth section (3), e.g. 2, 3, 4, 5, 6, 7, 8, 9, 10, or more growth sections (3), wherein each growth section (3) comprises the same multilayer cell assembly, cell aggregate, tissue, organoid, or organ. For example, each growth section (3) may comprise an organoid selected from the group consisting of a liver lobule, nephrons of kidney, the dermis and/or epidermis of skin, gut mucosa, Langerhans islets of pancreas, grey and white matter of brain cortex and cerebellum, and adult quiescence-promoting stem cell niches. Each growth section (3) may also comprise an organ selected from the group consisting of a micro-lung, micro-heart, micro-skin, micro-gut, micro-brain, micro-bone-marrow, micro-bone, micro-spleen, micro-pancreas, micro-testes, and micro-liver. It is also particularly more preferred that the three dimensional cell culture system (2) comprises more than one growth section (3), e.g. 2, 3, 4, 5, 6, 7, 8, 9, 10, or more growth sections (3), wherein each growth section (3) comprises another multilayer cell assembly, cell aggregate, tissue, organoid, or organ. For example, each growth section (3) may comprise an organoid (independently) selected from the group consisting of a liver lobule, nephrons of kidney, the dermis and/or epidermis of skin, gut mucosa, Langerhans islets of pancreas, grey and white matter of brain cortex and cerebellum, and adult quiescence-promoting stem cell niches. Each growth section (3) may also comprise an organ (independently) selected from the group consisting of a micro-lung, micro-heart, micro-skin, micro-gut, micro-brain, micro-bone-marrow, micro-cartilage, micro-bone, micro-spleen, micro-pancreas, micro-testes, and micro-liver. Thus, in a most preferred embodiment, the three dimensional cell culture system (2) is a multi-cell aggregate, multi-tissue, multi-organoid, and/or multi-organ three dimensional cell culture system (2), e.g. a multi-organoid three dimensional cell culture system (2), wherein the organoids are (independently) selected from the group consisting of liver lobule, nephrons of kidney, the dermis and/or epidermis of skin, gut mucosa, Langerhans islets of pancreas, grey and white matter of brain cortex and cerebellum, and adult quiescence-promoting stem cell niches, or a multi-organ three dimensional cell culture system (2), wherein the organs are (independently) selected from the group consisting of a micro-lung, micro-heart, micro-skin, micro-gut, micro-brain, micro-bone-marrow, micro-cartilage, micro-bone, micro-spleen, micro-pancreas, micro-testes, and micro-liver. A multi-organ three dimensional cell culture system (2) may also be designated as multi-organ-chip (MOC).

Also disclosed is an optical sensor device (1) configured to carry out the method for determining and/or monitoring at least one condition, preferably two or three conditions, of a three dimensional cell culture system (2) comprising at least one growth section (3) or in a three dimensional cell culture system (2) comprising at least one growth section (3), wherein the at least one condition, preferably two or three conditions, is (are) selected from the group consisting of
(i) physiological condition,
(ii) vitality, and
(iii) metabolism status,
wherein the physiological condition is determined using erythrocytes as detectors for said condition, the vitality is determined and/or monitored by measuring living cell fluorescent dyes, and the metabolism status is determined and/or monitored by measuring the autofluorescence of NADH and/or FAD, and/or by determining the NADH/NAD⁺ and/or NADH/FAD ratio according to the present invention.

Preferably, the (two or three) conditions determined and/or monitored are (i) the physiological condition and the vitality, (ii) the physiological condition and the metabolism status, (iii) the vitality and the metabolism status, or (iv) the physiological condition, the vitality and the metabolism status, wherein the physiological condition is determined using erythrocytes as detectors for said condition, the vitality is determined and/or monitored by measuring living cell fluorescent dyes, and the metabolism status is determined and/or monitored by measuring the autofluorescence of NADH and/or FAD, and/or by determining the NADH/NAD⁺ and/or NADH/FAD ratio.

As to the definition of the terms "cell vitality", "cell metabolism", "physiological condition", it is referred to the present invention as defined herein.

The physiological condition is preferably the flow rate, physiological osmolality, and/or oxygen consumption in the three dimensional cell culture system (2) or of the three dimensional cell culture system (2). It is preferred that erythrocytes are used as detectors for determining the flow rate, physiological osmolality, and/or oxygen consumption in the three dimensional cell culture system (2) or of the three dimensional cell culture system (2), e.g. (i) the flow rate and physiological osmolality, (ii) the flow rate and oxygen consumption, (iii) the physiological osmolality and the oxygen consumption, or (iv) the flow rate, physiological osmolality and oxygen consumption.

As to the preferred embodiments of the method, it is referred to the present invention as defined herein.

The optical sensor device (1) is preferably configured to carry out the above mentioned method for determining and/or monitoring the above mentioned at least one condition, e.g. at least one, two, or three condition(s), contact-free and non-invasive, particularly to avoid the direct intervention with the environment of the three dimensional cell culture system (2), e.g. the sterile, self-contained, and/or circulation three dimensional cell culture system (2). Thus, the optical sensor device (1) is preferably configured to allow the measurement from the outside of the three dimensional cell culture system (2), for example, directly through the body of the three dimensional cell culture system (2) which may be translucent and, for example, without direct contact between the optical sensor device (1) and the three dimensional cell culture system (2), e.g. without insertion of the optical sensor device (1) in the three dimensional cell culture system (2), particularly in the cell aggregate(s), tissue(s), organoid(s), and/or organ(s) comprised in the at least one growth section (3).

Further, said optical sensor device (1) preferably does not require the manipulation and/or modification of the three dimensional cell culture system (2), e.g. the sterile, self-contained, and/or circulation three dimensional cell culture system (2), in order to carry out the above method for determining and/or monitoring the above mentioned at least one condition, e.g. at least one, two, or three condition(s). Such a handling preferably reduces the risk for contamination and maintains the sterile environment in the three dimensional cell culture system (2). This is particularly important as the three dimensional cell culture system (2) is highly complex and fragile.

Furthermore, the optical sensor device (1) preferably allows the automated conduction of the above method, particularly for continuous monitoring of the above mentioned at least one condition, e.g. at least one, two or three condition(s).

Moreover, the optical sensor device (1) has the advantage that it allows the conduction of the above described methods in a three dimensional cell culture system (2) and not only in a monolayer of cells. As its name applies, a three dimensional cell culture system (2) is a three dimensional (3D) assembly of cells, e.g. a cell aggregate, a tissue, an organoid, or an organ. As mentioned above, the three dimensional (3D) assembly of cells, e.g. a cell aggregate, a tissue, an organoid, or an organ, is preferably comprised in at least one growth section (3) being part of the three dimensional cell culture system (2). Said growth section (3) or at least a part of said growth section (3) comprising, e.g. a cell aggregate, a tissue, an organoid, or an organ, is preferably measured with the optical sensor device (1). Particularly, the optical sensor device (1) allows the conduction of the above described methods in an area of at least 50x50x50 µm, 100x100x100 µm, 150x150x150 µm, 200x200x200 µm, 250x250x250 µm, 300x300x300 µm, 350x350x350 µm, 400x400x400 µm, 450x450x450 µm, or 500x500x500 µm, more particularly in an area of at least 500x500x500 µm, e.g. the determination of the average fluorescence intensity of all living fluorescent dyes comprised in this area, the determination of the average fluorescence intensity of all cells comprised in this area, and the determination of the autofluorescence of NADH and/or FAD in this area. This area is preferably located within the three dimensional cell culture system (2) and not on the surface of said system, particularly in a depth of at least 50, 100, 150, 200, 250, 300, 350, 400, 450, or 500 µm, more particularly in a depth of at least 500 µm. More particularly, the optical sensor device (1) allows the conduction of the above described methods in an area of at least 50x50x50 µm, 100x100x100 µm, 150x150x150 µm, 200x200x200 µm, 250x250x250 µm, 300x300x300 µm, 350x350x350 µm, 400x400x400 µm, 450x450x450 µm, or 500x500x500 µm, most particularly in an area of at least 500x500x500 µm, and in a depth of at least 50, 100, 150, 200, 250, 300, 350, 400, 450, or 500 µm, most particularly in a depth of at least 500 µm. This is based on the fact that the optical sensor device (1) comprises at least one excitation fiber (4) which is configured to excitate cells or molecules, e.g. NADH and/or FAD, comprised in this cells located at these depths and/or in these areas, and at least one emission fiber (5) which is configured to detect all signals emitted by the cells or molecules, e.g. NADH and/or FAD, comprised in this cells located at these depths and/or in these areas. In contrast thereto, the prior art only allows the detection of fluorescent cells or fluorescent molecules, e.g. living fluorescent dyes, in a monolayer cell culture, e.g. using a fluorescent reader.

Accordingly, the optical sensor device (1) preferably comprises at least one light excitation fibre (4), e.g. at least 1, 2, 3, 4, 5, or more light excitation fibre(s) (4), and at least one light emission fibre (5), e.g. at least 1, 2, 3, 4, 5, or more light emission fibre(s) (5). A preferred optical sensor device (1) which comprises one light excitation fibre (4) and one light emission fibre (5) is shown in **Figure 1A****.**

The term "light excitation fibre (4)", as used herein, refers to a fibre configured to receive excitation light, particularly from a light source, and/or to supply at least a portion of the excitation light to the optic sample. The term "light emission fibre (5)", as used herein, refers to a fibre configured to receive light from the optic sample and/or to supply it to a light detector.

It is further preferred that the at least one light excitation fibre (4) is a Ultra Violet (UV) light excitation fibre, a VIS light excitation fibre, a NIR light excitation fibre, a UV/VIS light excitation fibre, a UV/NIR light excitation fibre, a VIS/NIR light excitation fibre, or a UV/VIS/NIR light excitation fibre. The term "UV light excitation fibre", as used herein, refers to a fibre configured to receive UV light, particularly from a light source such as from a laser or a light emitting diode (LED), and to supply at least a portion of the UV light to the optic sample. UV light is electromagnetic radiation with a wavelength shorter than that of visible light, but longer than X-rays, in the range of between about 10 nm to 400 nm, and energies from 3 eV to 124 eV. The UV light excitation fibre is more preferably configured to receive and to supply light at a wavelength of between about 320 and 350 nm, even more preferably between about 320 and 340 nm, and most preferably at a wavelength of 337 nm, e.g. at 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350 nm, which is a wavelength suitable to activate NADH. The respective light source is preferably a laser, more preferably a nitrogen laser (337 nm, 30 Hz), or a light emitting diode (LED). In another embodiment, the UV light excitation fibre is more preferably configured to receive and to supply light at a wavelength of between about 300 and 380 nm, even more preferably between about 330 and 360 nm, and most preferably at a wavelength of 330 nm, e.g. at 300, 305, 310, 315, 320, 325, 330, 335, 340, 345, 350, 355, 360, 365, 370, 375, or 380 nm, and/or the UV light excitation fibre is more preferably configured to receive and to supply light at a wavelength of between about 430 and 480 nm, even more preferably between about 440 and 460 nm, and most preferably at a wavelength of 450 nm, e.g. at 430, 435, 440, 445, 450, 455, 460, 465, 470, 475, or 480 nm, which is a wavelength suitable to activate FAD. The respective light source is preferably a laser, more preferably a nitrogen laser or a light emitting diode (LED). The UV light excitation fibre is most preferably configured to receive and to supply light at a wavelength of between about 320 and 350 nm, e.g. at a wavelength of 337 nm, which is a wavelength suitable to activate NADH, and to receive and supply light at a wavelength of between about 300 and 380 nm, e.g. at a wavelength of 330 nm, and/or at a wavelength of between about 430 and 480, e.g. at a wavelength of 450 nm, which is a wavelength suitable to activate FAD. As to the preferred light sources, it is referred to the above.

The term "VIS light excitation fibre" refers to a fibre configured to receive excitation light in the range of VIS spectra, particularly form a light source, and to supply at least a portion of the excitation light to the optic sample.

The term "NIR light excitation fibre" refers to a fibre configured to receive excitation light in the range of NIR spectra, particularly form a light source, and to supply at least a portion of the excitation light to the optic sample.

The term "UV/VIS light excitation fibre" refers to a fibre configured to receive excitation light in the range of UV and VIS spectra, particularly form a light source, and to supply at least a portion of the excitation light to the optic sample.

The term "UV/NIR light excitation fibre" refers to a fibre configured to receive excitation light in the range of UV and NIR spectra, particularly form a light source, and to supply at least a portion of the excitation light to the optic sample.

The term "VIS/NIR light excitation fibre" refers to a fibre configured to receive excitation light in the range of VIS and NIR spectra, particularly form a light source, and to supply at least a portion of the excitation light to the optic sample.

The term "UV/VIS/NIR light excitation fibre" refers to a fibre configured to receive excitation light in the range of UV, VIS and NIR spectra, particularly form a light source, and to supply at least a portion of the excitation light to the optic sample.

It is also, additionally or alternatively, preferred that the at least one light emission fibre (5) is selected from the group consisting of
(i) an NADH emission fibre (6),
(ii) a FAD emission fibre (7),
(iii) a fluorescence emission fibre (8), and
(iv) an infrared emission fibre (9).

A preferred optical sensor device (1) comprises a light excitation fibre (4), an NADH emission fibre (6), a FAD emission fibre (7), a fluorescence emission fibre (8), and an infrared emission fibre (9) is shown in **Figure 1B****.**

More preferably, the optical sensor device (1) comprises
(i) an NADH emission fibre (6) and a FAD emission fibre (7),
(ii) an NADH emission fibre (6) and a fluorescence emission (8),
(iii) an NADH emission fibre (6) an infrared emission fibre (9),
(iv) a FAD emission fibre (7) and a fluorescence emission fibre (8),
(v) a FAD emission fibre (7) and an infrared emission fibre (9)
(vi) a fluorescence emission fibre (8) and an infrared emission fibre (9),
(vi) an NADH emission fibre (6), a FAD emission fibre (7), and a fluorescence emission fibre (8),
(vii) an NADH emission fibre (6), a fluorescence emission fibre (8), and an infrared emission fibre (9),
(viii) an NADH emission fibre (6), a FAD emission fibre (7), and an infrared emission fibre (9),
(ix) an NADH emission fibre (6), a FAD emission fibre (7), and an infrared emission fibre (9),
(x) a FAD emission fibre (7), a fluorescence emission fibre (8), and an infrared emission fibre (9), and
(xi) an NADH emission fibre (6), a FAD emission fibre (7), a fluorescence emission fibre (8), and an infrared emission fibre (9).

The optical sensor device (1) is most preferably a combination device comprising at least one light excitation fibre (4), an NADH emission fibre (6), and a FAD emission fibre (7).

The term "NADH emission fibre (6)", as used herein, refers to a fibre configured to receive NADH light (or NADH autofluorescence) from the optic sample and to supply it to a light detector. The NADH emission fibre (6) is more preferably configured to receive and supply light (or autofluorescence) at a wavelength of between about 400 and 490 nm, even more preferably between about 420 and 460 nm, and most preferably at a wavelength of 450 nm or 460 nm, e.g. at 400, 405, 410, 415, 420, 425, 430, 435, 440, 445, 450, 455, 460, 465, 470, 475, 480, 485, or 490 nm. As already mentioned above, NADH can be activated at a wavelength of between about 320 and 350 nm, e.g. at 337 nm, and the autofluorescence of NADH can be measured at a wavelength of between about 400 and 490 nm, e.g. at 450 or 460 nm.

The term "FAD emission fibre (7)", as used herein, refers to a fibre configured to receive FAD light (or FAD autofluorescence) from the optic sample and to supply it to a light detector. The FAD emission fibre (7) is more preferably configured to receive and supply light (or autofluorescence) at a wavelength of between about 500 and 560 nm, even more preferably between about 520 and 540 nm, and most preferably at a wavelength of 520 or 530 nm, e.g. at 500, 505, 510, 515, 520, 525, 530, 535, 540, 545, 550, or 560 nm. As already mentioned above, FAD can be activated at a wavelength of between about 300 and 380 nm or at a wavelength of between about 430 and 480 nm and the autofluorescence of FAD can be measured at a wavelength of between about 500 and 560 nm, e.g. at 520 or 530 nm.

The term "fluorescence emission fibre (8)", as used herein, refers to a fibre configured to receive fluorescent light from the optic sample and to supply it to a light detector. Fluorescence is the emission of light by an optical sample that has absorbed light or other electromagnetic radiation. It is a form of luminescence. In most cases, emitted light has a longer wavelength, and therefore lower energy, than the absorbed radiation. The emission spectrum is dominated by a short-wave UV line at 254 nm, accompanied by visible light emission at 436 nm (blue), 546 nm (green) and 579 nm (yellow-orange). The fluorescence emission fibre (8) is more preferably configured to receive light at a wavelength of between 500 and 620 nm, preferably at a wavelength of 517 or 602 nm.

The term "infrared (IR) emission fibre (9)", as used herein, refers to a fibre configured to receive infrared (IR) light from the optic sample and to supply it to a light detector. Infrared (IR) light is electromagnetic radiation with longer wavelengths than those of visible light, extending from the nominal red edge of the visible spectrum at 0.74 micrometers (µm) to 300 µm. This range of wavelengths corresponds to a frequency range of approximately 1 to 400 THz, and includes most of the thermal radiation emitted by objects near room temperature. The IR emission fibre (9) is most preferably configured to receive light at near IR spectra.

The light source is preferably a laser or a light emitting diode (LED). More preferably, the laser is a nitrogen laser, argon laser or TiSa laser.

The light detector is preferably a spectrometer, more preferably a UV spectrometer, VIS spectrometer, NIR spectrometer, UV/VIS spectrometer, UV/NIR spectrometer, VIS/NIR spectrometer, or a UV/VIS/NIR spectrometer, most preferably a UV/VIS/NIR spectrometer.

The optic sample is preferably the three dimensional cell culture system (2), particularly a part thereof, e.g. the at least one microfluidic channel, the at least one fluid reservoir, e.g. medium and/or blood reservoir, the at least one growth section (3) or at least a part thereof comprised in the three dimensional cell culture system (2). The at least growth section (3) preferably comprises a cell aggregate, a tissue, an organoid, or an organ.

It is further preferred that the at least one light excitation fibre (4) and that the at least one light emission fibre (5) have a round or square shape. The use of light excitation fibre(s) (4) and light emission fibre(s) (5) having a square shape has the advantage that a higher fiber density at a constant circumference of the optical sensor device (1) can be achieved.

It is further, preferably or alternatively, preferred that the round light excitation fibre has a smaller diameter than the round light emission fibre and/or that the square light excitation fibre has a smaller edge length than the square light emission fibre. This has the advantage that a comprehensive detection of the emission signal is possible. The transmission of the data is, thus, more precisely.

The (emission and/or excitation) fibres comprised in the optical sensor device (1) are preferably arranged in parallel. This, again, has the advantage that it increases the packaging density of optical sensor device (1).

It is also, preferably or alternatively, preferred that the fibres are coated with an isolation layer (10), preferably an aluminium or epoxy glue isolation layer, or the space between the fibres is filled with an isolation material (11), preferably an epoxy glue isolation material.

A preferred optical sensor device (1) comprising a light excitation fibre (4), an NADH emission fibre (6), a FAD emission fibre (7), a fluorescence emission fibre (8), and an infrared emission fibre (9), wherein each fibre is coated with an isolation layer (10) is shown in **Figure 1C****.**

Further, a preferred optical sensor device (1) comprising a light excitation fibre (4), an NADH emission fibre (6), a FAD emission fibre (7), a fluorescence emission fibre (8), and an infrared emission fibre (9), wherein the space between the fibers is filled with an isolation material (11) is shown in **Figure 1D****.**

In a preferred embodiment, the optical device (1) is comprised in an apparatus (12) for the automatic operation of a three dimensional cell culture system (2) comprising at least one growth section (3).

Preferably, the apparatus (12) comprises a carrier platform (13) configured to receive at least one three dimensional cell culture system (2), more preferably between 1 and 500, even more preferably between 25 and 200, and most preferably between 25 and 150 three dimensional cell culture systems (2), e.g. at least 1, 10, 25, 50, 75, 100, 125, 150, 175, 200, 225, 250, 275, 300, 325, 350, 375, 400, 425, 450, 475, 500 or more three dimensional cell culture system(s) (2), comprising at least one growth section (3), more preferably between 1 and 2000 growth sections (3), e.g. 1, 2, 3, 4, 5, 6, 7, 8, 9, 10, 11, 12, 13, 14, 15, 16, 17, 18, 19, 20, 24, 30, 36, 48, 60, 72, 84, 96, 100, 108, 120, 132, 144, 156, 168, 180, 192, 204, 216, 228, 240, or more growth section(s) (3). In a preferred microtiterplate-like format, 2, 4, 6, 24, 96, 384 or even 1536 growth sections (3) are arranged in a 2:3 rectangular matrix on the three dimensional cell culture system (2).

Preferably, the carrier platform (13) comprises at least one opening, more preferably between 1 and 500, even more preferably between 25 and 200, and most preferably between 25 and 150 opening(s), e.g. at least 1, 10, 25, 50, 75, 100, 125, 150, 175, 200, 225, 250, 275, 300, 325, 350, 375, 400, 425, 450, 475, 500 or more opening(s), and/or at least one mounting element, more preferably between 1 and 500, even more preferably between 25 and 200, and most preferably between 25 and 150 mounting element(s), e.g. at least 1, 10, 25, 50, 75, 100, 125, 150, 175, 200, 225, 250, 275, 300, 325, 350, 375, 400, 425, 450, 475, 500 or more mounting element(s), configured to receive at least one three dimensional cell culture system (2).

As to the definition of the terms "three dimensional cell culture system (2)" and "growth section (3)" and as to the preferred embodiments of the "three dimensional cell culture system (2)" and the "growth section (3)", it is referred to the present invention as defined herein. It is particularly preferred that the at least one three dimensional cell culture system (2), particularly the body of the at least one three dimensional cell culture system (2), is made of a translucent material, e.g. of glass, such as calcium carbonate/sodium bicarbonate glass or quartz glass, or plastic. This allows the contact-free and non-invasive observation of the at least one three dimensional cell culture system (2), particularly with the optical sensor device (1). Particularly, it allows the analysis of the at least one three dimensional cell culture system (2) from the outside.

The carrier platform (13) is preferably temperable. This allows the easy adjustment of the culture temperature of the at least one three dimensional cell culture system (2), which may be received by the carrier platform (13). The culture temperature is preferably 37°C.

It is preferred that carrier platform (13), particularly comprising at least one mounting element configured to receive at least one three dimensional cell culture system (2), is also made of a translucent material, preferably glass, more preferably calcium carbonate/sodium bicarbonate glass or quartz glass, or plastic. The use of a translucent material for the carrier platform (13) facilitates the observation and/or analysis of the at least one three dimensional cell culture (2) system, which may be received by the carrier platform (13) with the optical sensor device (1).

It is preferred that the carrier platform (13) comprises a first surface (14) configured to receive at least one three dimensional cell culture system (2) and a second surface (15) positioned opposite to the first surface (14). It is particularly preferred that the optical sensor device (1) is located on the second surface (15) or on the first surface (14) of the carrier platform (13).

Preferably, the apparatus (12) further comprises a functional unit (16). It is particularly preferred that the functional unit (16) is located on the first surface (14) or on the second surface (15) of the carrier platform (13).

It is more particularly preferred that the functional unit (16) is located on a different surface than the optical sensor device (1). Most preferably, the optical sensor device (1) is located on the second surface (15) of the carrier platform (13), while the functional unit (16) is located on the first surface (14) of the carrier platform (13).

The functional unit (16) is preferably configured to allow the loading, exchange and/or application of material. Preferably, the functional unit (16) comprises an injection system (17), a rejection system (18), or a combination thereof (17/18). The injection system (17) is configured to allow the supply/application of material, e.g. fluid supply such as medium or blood supply, the application of substances such as nutrients, the application of test substances or living cell fluorescent dyes, e.g. via an injection port which is part of the three dimensional cell culture system (2), and/or the rejections system (18) is configured to allow the removal of material, e.g. fluid removal such as medium or blood removal, e.g. via an rejection port which is part of the three dimensional cell culture system (2). In a preferred embodiment, the injection and/or rejection system (17/18) is a pipette (e.g. a multi-channel pipette). Preferably, the three dimensional cell culture system (2) comprises a port, which allows the injection as well as the rejection of material, e.g. the removal/exchange of fluid such as medium and/or blood.

In a preferred embodiment, the apparatus (12) comprises at least one, preferably 2 or 3, holding element(s), e.g. single bridge(s) (26) and/or double bridge(s) (23), and/or a basis plate (22). It is preferred that the at least one, preferably 2 or 3, holding element(s), e.g. single bridge(s) (26) and/or double bridge(s) (23), is (are) connected with the basis plate (22).

Preferably, the optical sensor device (1) is arranged on such a holding element. The functional unit (16) may also be arranged on such a holding element. It is particularly preferred that the optical sensor device (1) and the functional unit (16) are arranged on such a holding element.

More preferably, the optical sensor device (1) is arranged on a single bridge (26) or double bridge (23). The functional unit (16) may also be arranged on a single bridge (26) or double bridge (23). The single bridge (26) preferably comprises a (first) footbridge element (27). It is particularly preferred that the optical sensor device (1) is positioned at the (first) footbridge element (27) of the single bridge (26). The functional unit (16) may also be positioned at the (first) footbridge element (27) of the single bridge (26). Preferably, the optical sensor device (1) and the functional unit (16) are arranged on different single bridges (26). In this case, the optical sensor device (1) and the functional unit (16) are preferably located opposite to each other.

The double bridge (23) preferably comprises a first footbridge element (24) and a second footbridge element (25). The optical sensor device (1) may be positioned at the first footbridge element (24) or at the second footbridge element (25). The functional unit (16) may also be positioned at the first footbridge element (24) or at the second footbridge element (25). It is more preferred that the optical sensor device (1) and the functional unit (16) are arranged on a double bridge (23). It is most preferred that the optical sensor device (1) is positioned at the second footbridge element (25) of the double bridge (23). Said double bridge (23) preferably further comprises the functional unit (16) which is positioned at the first footbridge element (24).

The above described first footbridge element (24) of the double bridge (23) is particularly located on the first surface (14) of the carrier platform (13) and the second footbridge element (25) of the double bridge (23) is particularly located on the second surface (15) of the carrier platform (13).

Further, the above described (first) footbridge element (27) of the single bridge (26) may be located on the first surface (14) of the carrier platform (13) or at the second surface (15) of the carrier platform (13).

It is further preferred that the carrier platform (13) is connected with the at least one, preferably 2 or 3, holding element(s), e.g. single bridge(s) (26) and/or double bridge(s) (23).

In a preferred embodiment, the apparatus (12) comprising the optical sensor device (1) contains at least one, preferably 2 or 3, movable holding elements, e.g. movable single bridge(s) (26) and/or movable double bridge(s) (23). Preferably, said at least one, preferably 2 or 3, holding element(s), e.g. single bridge(s) (26) and/or double bridge(s) (23), is (are) movable along the longitudinal axis of the basis plate (22) and/or carrier platform (13). More preferably, the optical sensor device (1) is positioned at the second footbridge element (25) of a movable double bridge (23). Said movable double bridge (23) preferably further comprises the functional unit (16) which is positioned at the first footbridge element (24). The carrier platform (13) and the basis plate (22) are preferably arranged in parallel.

In a further preferred embodiment, the optical sensor device (1) is movable along the longitudinal axis and/or lateral axis of the carrier platform (13) and/or basis plate (22). The functional unit (16) may also be movable along the longitudinal axis and/or lateral axis of the carrier platform (13) and/or basis plate (22).

In another preferred embodiment, the optical sensor device (1) is movable towards the carrier platform (13). Preferably, the optical device (1) which is arranged on the second footbridge element (25) of the double bridge (23) is movable towards the carrier platform (13). More preferably, the at least one light excitation fibre (4) and the at least one light emission fibre (5) comprised in said optical sensor device (1) are movable towards the carrier platform (13) or the fibre bundle of light excitation and light emission fibres (19) is movable towards the carrier platform (13). Most preferably, the at least one light excitation fibre (4) and the at least one light emission fibre (5) comprised in said optical sensor device (1) which is arranged on the second footbridge element (25) of the double bridge (23) are movable towards the carrier platform (13). The functional unit (16) may also be movable towards the carrier platform (13). The functional unit (16) which is arranged on the first footbridge element (24) of the double bridge (23) may be movable towards the carrier platform (13). More preferably, the injection system (17), rejection system (18), or a combination thereof (17/18) comprised in said functional unit (16) is movable towards the carrier platform (13). Most preferably, the injection system (17), rejection system (18), or a combination thereof (17/18) comprised in said functional unit (16) which is arranged on the first footbridge element (24) of the double bridge (23) is movable towards the carrier platform (13). The distance between the optical sensor device (1) and the carrier platform (13) amounts to between 0 and 1 mm, preferably between 0 and 500µm, more preferably 0 µm.

The carrier platform (13) of the apparatus (12) is preferably further configured to receive a media sample (20) and/or a test compound sample (21), e.g. to supply the at least one three dimensional cell culture system (2), particularly via the functional unit (16), e.g. injection system (17), with media, and/or to apply to the at least one three dimensionally cell culture system (2), particularly via the functional unit (16), e.g. injection system (17), with the test compound.

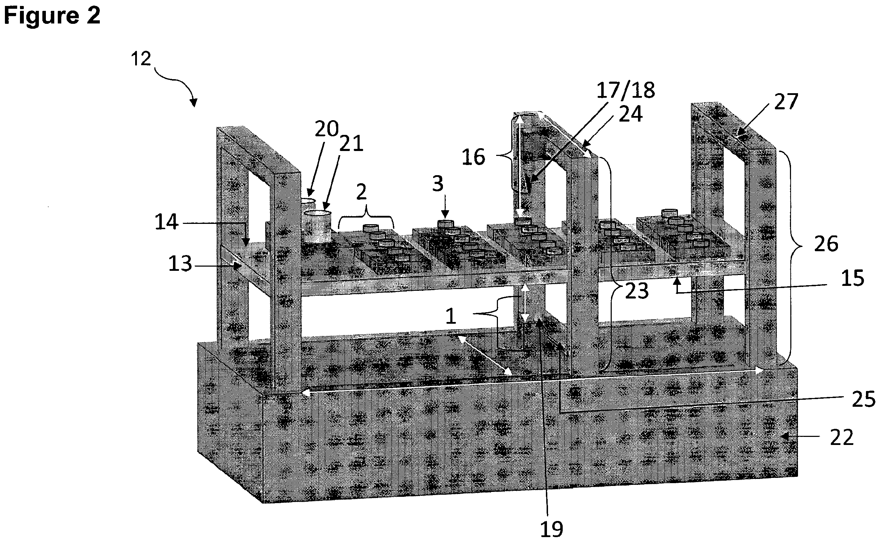

A preferred arrangement of the optical sensor device (1) disclosed herein in the apparatus (12) as described above is shown in **Figure 2****.**

Also disclosed is the use of the optical sensor device (1) disclosed herein for determining and/or monitoring at least one condition, preferably two or three conditions, in a three dimensional cell culture system (2) comprising at least one growth section (3), wherein the at least one condition, preferably two or three conditions, is (are) selected from the group consisting of
(i) physiological condition,
(ii) vitality, and
(iii) metabolism status,
wherein the physiological condition is determined using erythrocytes as detectors for said condition, the vitality is determined and/or monitored by measuring living cell fluorescent dyes, and the metabolism status is determined and/or monitored by measuring the autofluorescence of NADH and/or FAD, and/or by determining the NADH/NAD⁺ and/or NADH/FAD ratio. As to the determination or monitoring of the above mentioned at least one condition, preferably at least two or three conditions, in the three dimensional cell culture system (2) comprising at least one growth section (3), it is referred to the definitions and preferred embodiments as described in the context of the present invention.

Also disclosed is the use of the optical sensor device (1) disclosed herein for monitoring the effect of a test compound and/or for determining the efficacy, side-effects, biosafety, metabolites, mode of action or organ regeneration. As to the definition of the term "test compound" and as to the preferred embodiments of the "test compound", it is referred to the present invention as defined herein.

Also disclosed is an optical sensor device (1) which comprises at least one light excitation fibre (4), e.g. at least 1, 2, 3, 4, 5, or more light excitation fibre(s) (4), and at least one light emission fibre (5), e.g. at least 1, 2, 3, 4, 5, or more light emission fibre(s) (5). Preferably, the at least one light emission fibre (5) is selected from the group consisting of
(i) an NADH emission fibre (6),
(ii) a FAD emission fibre (7),
(iii) a fluorescence emission fibre (8), and
(iv) an infrared emission fibre (9).
Said optical sensor device (1) is preferably configured to carry out the method for determining and/or monitoring at least one condition in a three dimensional cell culture system (2) comprising at least one growth section (3), wherein the at least one condition is selected from the group consisting of
(i) physiological condition,
(ii) vitality, and
(iii) metabolism status,
wherein the physiological condition is determined using erythrocytes as detectors for said condition, the vitality is determined and/or monitored by measuring living cell fluorescent dyes, and the metabolism status is determined and/or monitored by measuring the autofluorescence of NADH and/or FAD, and/or by determining the NADH/NAD⁺ and/or NADH/FAD ratio according to the present invention.

As to the definition of the terms "light excitation fibre (4)", "light emission fibre (5)", "NADH emission fibre (6)", FAD emission fibre (7), "fluorescence emission fibre (8)" and "infrared emission fibre (9)" and as to other preferred embodiments of the optical sensor device (1), it is referred to the present disclosure. As to the method, which is preferably carried to with the optical sensor device (1) and as to the specific definitions used in this context, it is referred to the present invention as defined herein.

Also disclosed is an apparatus (12) for the automatic establishment and/or operation of a three dimensional cell culture system (2) comprising
(i) a carrier platform (13) configured to receive at least one three dimensional cell culture system (2) comprising at least one growth section (3),
(ii) at least one functional unit (16),
(iii) at least one optical sensor device (1),
(iv) at least one holding element (e.g. 1, 2, or 3 holding element(s)), preferably at least one double bridge (23) or at least two single bridges (26), and
(v) a basis plate (22),
wherein the at least one holding element, preferably at least one double bridge (23) or at least two single bridges (26), is (are) connected with the basis plate (22), the at least one functional unit (16) and/or the at least one optical sensor device (1) is arranged on the at least one holding element, preferably at least one double bridge (23) or at least two single bridges (26) (e.g. the at least one functional unit (16) is arranged on a single bridge (26) and the at least one optical sensor device (1) is arranged on another single bridge (26)), and the at least one functional unit (16) and the at least one optical sensor device (1) are located opposite to each other.

The specific arrangement of the at least one functional unit (16) and of the at least one optical sensor device (1) in the apparatus (12) has the advantage that it allows the simultaneous (i) supply of the at least one three dimensional cell culture system (2) with material, e.g. fluids such as media and/or blood, substances such as nutrients, test substances and/or chemicals such as living cell fluorescent dyes, via the functional unit (16), and (ii) monitoring of the at least one three dimensional cell culture system (2) via the optical sensor device (1).

Preferably, the carrier platform (13) comprises at least one opening, more preferably between 1 and 500, even more preferably between 25 and 200, and most preferably between 25 and 150 opening(s), e.g. at least 1, 10, 25, 50, 75, 100, 125, 150, 175, 200, 225, 250, 275, 300, 325, 350, 375, 400, 425, 450, 475, 500 or more opening(s), and/or at least one mounting element, more preferably between 1 and 500, even more preferably between 25 and 200, and most preferably between 25 and 150 mounting element(s), e.g. at least 1, 10, 25, 50, 75, 100, 125, 150, 175, 200, 225, 250, 275, 300, 325, 350, 375, 400, 425, 450, 475, 500 or more mounting element(s), configured to receive a three dimensional cell culture system (2).

As to the definition of the terms "three dimensional cell culture system (2)" and "growth section (3)" and as to the preferred embodiments of the "three dimensional cell culture system (2)" and the "growth section (3)", it is referred to the present invention as defined herein. It is particularly preferred that the at least one three dimensional cell culture system (2), particularly the body of the at least one three dimensional cell culture system (2), is made of a translucent material, e.g. of glass, such as calcium carbonate/sodium bicarbonate glass or quartz glass, or plastic. This allows the contact-free and non-invasive observation of the at least one three dimensional cell culture system (2), particularly with the optical sensor device (1). Particularly, it allows the analysis of the at least one three dimensional cell culture system (2) from the outside.

The carrier platform (13) is preferably temperable. This allows the easy adjustment of the culture temperature of the at least one three dimensional cell culture system (2), which may be received by the carrier platform (13). The culture temperature is preferably 37°C.

It is preferred that carrier platform (13), particularly comprising at least one mounting element configured to receive at least one three dimensional cell culture system (2), is also made of a translucent material, preferably glass, more preferably calcium carbonate/sodium bicarbonate glass or quartz glass, or plastic. The use of a translucent material for the carrier platform (13) facilitates the observation and/or analysis of the at least one three dimensional cell culture (2) system, which may be received by the carrier platform (13) from the outside, particularly with the optical sensor device (1).

The carrier platform (13) preferably comprises a first surface (14) configured to receive the at least one three dimensional cell culture system (2) and a second surface (15) positioned opposite to the first surface (14). The at least one functional unit (16) may be located on the first surface (14) or on the second surface (15) of the carrier platform (13). It is particularly preferred that the at least one functional unit (16) is located on the first surface (14) of the carrier platform (13). The at least one optical sensor device (1) may be located on the second surface (15) or on the first surface (14) of the carrier platform (13). It particularly preferred that the at least one optical sensor device (1) is located on the second surface (15) of the carrier platform (13). It is particularly more preferred that the least one functional unit (16) is located on the first surface (14) of the carrier platform (13) and that the at least one optical sensor device (1) is located on the second surface (15) of the carrier platform (13). The at least one functional unit (16) is preferably configured to allow the loading, exchange and/or application of material. Preferably, the at least one functional unit (16) comprises an injection system (17), a rejection system (18), or a combination thereof (17/18). The injection system (17) is configured to allow the supply/application of material, e.g. fluid supply such as medium or blood supply, the application of substances such as nutrients, the application of test substances or living cell fluorescent dyes, e.g. via an injection port which is part of the three dimensional cell culture system (2), and/or the rejections system (18) is configured to allow the removal of material, e.g. fluid removal such as medium or blood removal, e.g. via an rejection port which is part of the three dimensional cell culture system (2). In a preferred embodiment, the injection and/or rejection system (17/18) is a pipette (e.g. a multi-channel pipette). Preferably, the three dimensional cell culture system (2) comprises a port, which allows the injection as well as the rejection of material, e.g. the removal/exchange of fluid such as medium and/or blood.

It is preferred that the at least one optical sensor device (1) is configured to allow the contract-free, non-invasive monitoring of the at least one three dimensional cell culture system (2).

Preferably, the carrier platform (13) is attached to the at least one holding element, e.g. 1, 2, or 3 holding element(s), and/or the basis plate (22) is attached to the at least one holding element, e.g. 1, 2, or 3 holding element(s).

It is preferred that the at least one functional unit (16) or the at least one optical sensor device (1) is arranged on a single bridge (26). For example, the at least one functional unit (16) is arranged on a single bridge (26) and the at least one optical sensor device (1) is arranged on another single bridge (26), provided that the at least one functional unit (16) and that the at least one optical sensor device (1) are located opposite to each other.

It is also preferred that the at least one functional unit (16) or the at least one optical sensor device (1) is arranged on a double bridge (23). For example, the at least one functional unit (16) is arranged on a double bridge (23) and the at least one optical sensor device (1) is arranged on another double bridge (23), provided that the at least one functional unit (16) and that the at least one optical sensor device (1) are located opposite to each other.

It is more preferred that the at least one functional unit (16) and that the at least one optical sensor device (1) are arranged on a double bridge (23).

It is particularly preferred that the single bridge (26) comprises a (first) food bridge element (27). The functional unit (16) may be positioned on the (first) footbridge element (27) of the single bridge (26) and/or the optical sensor device (1) may be positioned on the (first) bridge element (27) of the single bridge (26). For example, the at least one functional unit (16) is positioned on the (first) footbridge element (27) of the single bridge (26) and the at least one optical sensor device (1) is positioned on the (first) footbridge element (27) of another single bridge (26), provided that the at least one functional unit (16) and that the at least one optical sensor device (1) are located opposite to each other. It is particularly more preferred that the double bridge (23) comprises a first footbridge element (24) and a second footbridge element (25). The functional unit (16) may be positioned on the first footbridge element (24) and the optical sensor device (1) may be positioned on the second footbridge element (25) of the double bridge (23), or the functional unit (16) may be positioned on the second footbridge element (25) and the optical sensor device (1) may be positioned on the first footbridge element (24) of the double bridge (23). It is particularly most preferred that the at least one functional unit (16) is positioned on the first footbridge element (24) and that the optical sensor device (1) is positioned on the second footbridge element (25) of the double bridge (23).

The above described first footbridge element (24) of the double bridge (23) is particularly located on the first surface (14) of the carrier platform (13) and the second footbridge element (25) of the double bridge (23) is particularly located on the second surface (15) of the carrier platform (13).

Further, the above described (first) footbridge element (27) of the single bridge (26) may be located on the first surface (14) of the carrier platform (13) or at the second surface (15) of the carrier platform (13).

It is further preferred that the carrier platform (13) and the basis plate (22) are arranged in parallel. It is further particularly preferred that the at least one functional unit (16) and/or the at least one optical sensor device (1) are movable relative to each other. This allows the separated or commonly piloting of the at least one three dimensional cell culture system (2), e.g. in order to change fluid, e.g. medium and/or blood, in the three dimensional cell culture system (2) via the functional unit (16), to supply a test compound to the three dimensional cell culture system (2) via the functional unit (16) and to monitor the effect of the test compound on the three dimensional cell culture system (2) via the optical sensor device (1), particularly at the same time point during culture.

It is particularly preferred that the carrier platform (13) is movable along the longitudinal axis of the basis plate (22) and/or the holding element, e.g. double bridge (23) or single bridge (26). It is also particularly preferred that the functional unit (16) and/or the optical sensor device (1) are movable along the lateral axis of the carrier platform (13). It is further particularly preferred that the optical sensor device (1) is movable towards the carrier platform (13) and/or the functional unit (16) is movable towards the carrier platform (13). It is more particularly preferred that the fibre bundle of light excitation and light emission fibres (19) comprised in the optical sensor device (1) is movable towards the carrier platform (13) and/or that the combination of the injection/rejection system (17/18) comprised in the functional unit (16) is movable towards the carrier platform (13). As mentioned above, the optical sensor device (1) allows the contract-free and non-invasive monitoring of the at least one three dimensional cell culture. Thus, said optical sensor device (1) can be brought close to the at least one three dimensional cell culture system (2) but it is never inserted or introduced in said system. Preferably, the distance between the optical sensor device (1) and the carrier platform (13) amounts to between 0 and 1mm, preferably between 0 and 500µm, more preferably 0 µm.

The apparatus (12) is preferably further equipped with at least one control unit which allows the operation of said apparatus (12) in an automatic manner. Such an apparatus allows the automated establishment and/or operation of at least one three dimensional cell culture systems (2), more preferably between 1 and 500, even more preferably between 25 and 200, and most preferably between 25 and 150 three dimensional cell culture systems (2), e.g. at least 1, 10, 25, 50, 75, 100, 125, 150, 175, 200, 225, 250, 275, 300, 325, 350, 375, 400, 425, 450, 475, 500 or more three dimensional cell culture system(s) (2). The automated establishment and/or operation of the three dimensional cell culture systems (2) is economic as well as time-saving in contrast to the manual operation of the three dimensional cell culture systems (2).

The automatic establishment of the three dimensional cell culture system (2) preferably comprises the steps of (i) seeding cells such as endothelial cells, particularly via the at least one functional unit (16) of the apparatus (12), into the three dimensional cell culture system (2), particularly via the injection port of said system, and/or (ii) incubating the three dimensional cell culture system (2), at least until a cell culture, tissue culture, or organoid has formed in the three dimensional cell culture system (2). Optionally, the automatic establishment of the test system (11) further comprises the step of injecting whole blood or medium, particularly via the at least one functional unit (16) of the apparatus (12), into the three dimensional cell culture system (2), particularly via the injection port of said system.

The automatic operation of the three dimensional cell culture system (2) preferably comprises the steps of fluid exchange, e.g. blood and/or medium exchange, particularly via the at least one functional unit (16) of the apparatus (12), the conduction of standardised toxicity tests, i.e. tests to study the toxic effect of one or more test substances upon the at least one three dimensional cell culture system (2), e.g. by applying the test compound, particularly via the injection port, to the three dimensional cell culture system (2), and the conduction of the methods according to the invention, particularly via the optical sensor device (1) of the apparatus (12).

The carrier platform (13) of the apparatus (12) is preferably further configured to receive a media sample (20) and/or a test compound sample (21), e.g. to supply the at least one three dimensional cell culture system (2), particularly via the functional unit (16), e.g. injection system (17), with media, and/or to apply to the at least one three dimensionally cell culture system (2), particularly via the functional unit (16), e.g. injection system (17), with the test compound.

A preferred apparatus (12) for the automated establishment and/or operation of a three dimensional cell culture system (2) disclosed herein comprising an optical sensor device (1) is shown in **Figure 2****.**

### LIST OF REFERENCE NUMBERS

- (1): optical sensor device
- (2): three dimensional cell culture system
- (3): growth section
- (4): light excitation fibre
- (5): light emission fibre
- (6): NADH emission fibre
- (7): FAD emission fibre
- (8): fluorescence emission fibre
- (9): infrared emission fibre
- (10): isolation layer
- (11): isolation material
- (12): apparatus
- (13): carrier platform
- (14): first surface of the carrier platform
- (15): second surface of the carrier platform
- (16): functional unit
- (17): injection system
- (18): rejection system
- (19): fibre bundle of light excitation and light emission fires
- (20): media sample
- (21): test compound sample
- (22): basis plate
- (23): double bridge
- (24): first footbridge element
- (25): second footbridge element
- (26): single bridge
- (27): footbridge element

### BRIEF DESCRIPTION OF THE FIGURES

**Figure 1****:** Cross sections of optical sensor devices (1). **(A)** Cross section of an optical sensor device (1) which comprises one light excitation fibre (4) and one light emission fibre (5); **(B)** cross section of an optical sensor device (1) which comprises a light excitation fibre (4), an NADH emission fibre (6), a FAD emission fibre (7), a fluorescence emission fibre (8), and an infrared emission fibre (9); **(C)** cross section of an optical sensor device (1) comprising a light excitation fibre (4), an NADH emission fibre (6), a FAD emission fibre (7), a fluorescence emission fibre (8), and an infrared emission fibre (9), wherein each fibre is coated with an isolation layer (10); and **(D)** cross section of an optical sensor device (1) comprising a light excitation fibre (4), an NADH emission fibre (6), a FAD emission fibre (7), a fluorescence emission fibre (8), and an infrared emission fibre (9), wherein the space between the fibers is filled with an isolation material (11).

**Figure 2****:** Shows a drawing of an apparatus (12) for the automatic establishment and/or operation of a three dimensional cell culture system (2) comprising (i) a carrier platform (13) with carries three dimensional cell culture systems (2) comprising one growth section (3), (ii) one functional unit (16) comprising a combination of an injection/rejection system (17/18), (iii) one optical sensor device (1) with fibre bundles of light excitation and light emission fibres (19), (iv) one double bridge (23) and two single bridges (26), and (v) a basis plate (22), wherein the one double bridge (23) and the two single bridges (26) are connected with the basis plate (22), the one functional unit (16) comprising a combination of an injection/rejection system (17/18) and/or the one optical sensor device (1) are arranged opposite to each other on the double bridge (23). The carrier platform (13) is temperable and made of a translucent material. The three dimensional cell culture systems (2) are also made of a translucent material. The carrier platform (13) comprises a first surface (14) which carries the three dimensional cell culture systems (2) and a second surface (15) positioned opposite to the first surface (14). The double bridge (23) comprises a first footbridge element (24) and a second footbridge element (25). Said first footbridge element (24) of the double bridge (23) is located on the first surface (14) of the carrier platform (13) and the second footbridge element (25) of the double bridge (23) is located on the second surface (15) of the carrier platform (13). The one functional unit (16) comprising a combination of an injection/rejection system (17/18) is positioned on the first footbridge element (24) and the optical sensor device (1) is positioned on the second footbridge element (25) of the double bridge (23). The two single bridges (26) comprise a footbridge element (27). Said footbridge element (27) of the single bridge (26) is located on the first surface (14) of the carrier platform (13). The carrier platform (13) is further attached to the one double bridge (23) and two single bridges (26). The carrier platform (13) of the apparatus (12) further carries a media sample (20) and a test compound sample (21), e.g. to supply the three dimensional cell culture systems (2) via the injection system (17) of the functional unit (16) with media, and/or to apply to the three dimensionally cell culture systems (2) via the injection system (17) of the functional unit (16) one or more test compound(s). The carrier platform (13) and the basis plate (22) are arranged in parallel. The carrier platform (13) is movable along the longitudinal axis of the basis plate (22). The one functional unit (16) comprising a combination of an injection/rejection system (17/18) and the one optical sensor device (1) are movable relative to each other. This allows the separated or commonly piloting of the three dimensional cell culture systems (2), e.g. in order to change fluid, e.g. medium and/or blood, in the three dimensional cell culture system (2) via the injection system (17) of the functional unit (16), to supply one or more test compound(s) to the three dimensional cell culture systems (2) via the injection system (17) of the functional unit (16) and to monitor the effect of the one or more test compound(s) on the three dimensional cell culture systems (2) via the optical sensor device (1), particularly at the same time point during culture. The optical sensor device (1) is movable along the lateral axis of the carrier platform (13) and the functional unit (16) comprising a combination of an injection/rejection system (17/18) is movable along the lateral axis of the carrier platform (13). The fibre bundle of light excitation and light emission fibres (19) comprised in the optical sensor device (1) is further movable towards the carrier platform (13) and the combination of the injection/rejection system (17/18) comprised in the functional unit (16) is movable towards the carrier platform (13). The optical sensor device (1) allows the contract-free and non-invasive monitoring of the three dimensional cell culture made of a translucent material which is carried from the carrier platform (13) also made of a translucent material. Said optical sensor device (1) can be brought close to the three dimensional cell culture systems (2) but it is never inserted or introduced in said system. The distance between the optical sensor device (1) and the carrier platform (13) may amount to between 0 and 1 mm or between 0 and 500µm. White colored double arrows in the drawing represent the above described moving directions.

### BRIEF DESCRIPTION OF THE EXAMPLES

### Experiment 1:

The following experiment will be used to evaluate the NADH autofluorescence of different cell aggregates, organoids or organs in a Multi-Organ-Chip (MOC). Therefore, a MOC will be created and mounted in a support. The MOC will be rinsed and filled with media. Afterwards, different cell types in different concentrations are going to be placed inside the growth sections on the MOC. Said different cell types will develop to different cell aggregates, organoids or organs during continuous culture. The establishment of such a MOC is described, for example, in WO 2009/146911 A2 (see particularly page 3, lines 6 to 20, page 37, page 1 to page 38, line 2, and page 38, line 24 to page 39, line 15, and in claims 33, and 38 to 42) or WO 2012/016711 A1 (see particularly page 3, lines 14 to 21, on page 15, line 1 to page 16, line 14, page 19, lines 10 to 33, page 20, lines 5 to 23, and page 22, line 1 to page 23, line 8 and in claims 18 to 20). To measure NADH autofluorescence in said MOC, an optical sensor device comprising an excitation fiber and an emission fiber will be used. The excitation fiber is connected to a light source (e.g. a laser or LED) which emits light at 337nm. The excitation fiber receives said light and supplies it to the different cell aggregates, organoids or organs comprised in the MOC. NADH can be activated at a wavelength of 337 nm. The emission fiber receives the autofluorescence of NADH in the different cell aggregates, organoids or organs and supplies it to a light detector (e.g. a spectrometer). The detector evaluates the emission of NADH at 450 nm wavelength. The emission fiber is attached to the bottom glass slide of the MOC. The optical sensor device is used to excite NADH autofluorescence at 337nm within the chip and to detect NADH emissions at 450 nm wavelength. Potential bandpass filters might be used to purify the detected emission. The signal is going to be measured at static culture and in a perfused MOC. Within the experiment also several different fibers and detectors will be used.

### Experiment 2:

The following experiment will be used to evaluate the FAD autofluorescence of different cell aggregates, organoids or organs in a Multi-Organ-Chip (MOC). Therefore, a MOC will be created and mounted in a support. The MOC will be rinsed and filled with media. Afterwards, different cell types in different concentrations are going to be placed inside the growth sections on the MOC. Said different cell types will develop to different cell aggregates, organoids or organs during continuous culture. The establishment of such a MOC is described, for example, in WO 2009/146911 A2 (see particularly page 3, lines 6 to 20, page 37, page 1 to page 38, line 2, and page 38, line 24 to page 39, line 15, and in claims 33, and 38 to 42) or WO 2012/016711 A1 (see particularly page 3, lines 14 to 21, on page 15, line 1 to page 16, line 14, page 19, lines 10 to 33, page 20, lines 5 to 23, and page 22, line 1 to page 23, line 8 and in claims 18 to 20). To measure FAD autofluorescence in said MOC, an optical sensor device comprising an excitation fiber and an emission fiber will be used. The excitation fiber is connected to a light source (e.g. a laser or LED) which emits light at 450 nm. The excitation fiber receives said light and supplies it to the different cell aggregates, organoids or organs comprised in the MOC. FAD can be activated at a wavelength of 450 nm. The emission fiber receives the autofluorescence of FAD in the different cell aggregates, organoids or organs and supplies it to a light detector (e.g. a spectrometer). The detector evaluates the emission of FAD at 520 nm wavelength. The emission fiber is attached to the bottom glass slide of the MOC. The optical sensor device is used to excite FAD autofluorescence at 450 nm within the chip and to detect FAD emissions at 520 nm wavelength. Potential bandpass filters might be used to purify the detected emission. The signal is going to be measured at static culture and in a perfused MOC. Within the experiment also several different fibers and detectors will be used.

### Experiment 3:

To gain more detail about the oxygen consumption of organs cultured in the growth sections, the haemoglobin saturation of erythrocytes will be measured. On the basis of the haemoglobin saturation, the oxygen consumption will be determined. Therefore, MOCs will be produced and placed in a support as described above. Instead of cell culture media, the chip is going to be filled with human blood and perfused. Afterwards, the haemoglobin saturation is measured before and after the growth section on the chip. Between these two points/positions, the media has to perfuse the growth section filled with organoids, organs, or cell aggregates, etc. With the help of an infrared light source and a detector, the haemoglobin saturation can be measured. On the basis of the haemoglobin saturation at the different points/positions, the oxygen consumption will be calculated. The potential drop in haemoglobin saturation is most likely due to the consumption of oxygen within the growth section. The amount of oxygen consumed further relates to the metabolic activity of the cell.

## Claims

1. An *in vitro* method for determining and/or monitoring at least one condition of/in a three dimensional cell culture system (2) comprising at least one growth section (3), wherein the at least one condition comprises a physiological condition and further comprises a condition selected from the group consisting of
(i) vitality, and
(ii) metabolism status,
wherein the physiological condition is determined using erythrocytes as detectors for said condition by determining the flow rate, physiological osmolality, and/or oxygen consumption in the three dimensional cell culture system (2), the vitality is determined and/or monitored by measuring living cell fluorescent dyes, and the metabolism status is determined and/or monitored by measuring the autofluorescence of NADH and/or FAD, and/or by determining the NADH/NAD⁺ and/or NADH/FAD ratio.

2. The method of claim 1, wherein the method for determining the flow rate in the three dimensional cell culture system (2) using erythrocytes as detectors comprises the steps of:
(i) providing a perfusable three dimensional cell culture system (2) comprising at least one growth section (3),
(ii) taking pictures of erythrocytes in said cell culture system at least at two different time points,
(iii) selecting at least one erythrocyte and determining the position of the at least one erythrocyte on the at least two pictures,
(iv) assigning a motion vector to the at least one erythrocyte representing the positional change of said at least one erythrocyte on the at least two pictures, and
(v) determining the flow rate in said cell culture system on the basis of the motion vector.

3. The method of claims 1 to 2, wherein the method for determining the physiological osmolality in the three dimensional cell culture system (2) using erythrocytes as detectors comprises the steps of:
(i) providing the three dimensional cell culture system (2) comprising at least one growth section (3), and
(ii) determining the shape of at least one erythrocyte in said cell culture system at a time point during culturing of said cell culture system.

4. The method of claim 3, wherein
(i) a spherical shape of the at least one erythrocyte indicates that the physiological osmolality is decreased (hypotonic condition), or
(ii) a crenate shape of the at least one erythrocyte indicates that the physiological osmolality is increased (hypertonic condition), or
(iii) a biconcave shape of the at least one erythrocyte indicates that the physiological osmolality is stable (isotonic condition).

5. The method of claims 1 to 4, wherein the method for determining the oxygen consumption in the three dimensional cell culture system (2) using erythrocytes as detectors comprises the steps of:
(i) providing the three dimensional cell culture system (2) comprising at least one growth section (3),
(ii) determining an average haemoglobin saturation of erythrocytes at a first position and at a second position in said cell culture system, and
(iii) calculating the oxygen consumption in said cell culture system on the basis of the average haemoglobin saturation at the different positions.

6. The method of claims 3 to 5, wherein the three dimensional cell culture system (2) is perfusable.

7. The method of claim 1, wherein the method for determining and/or monitoring the vitality of the three dimensional cell culture system (2) by measuring living cell fluorescent dyes comprises the steps of:
(i) providing the three dimensional cell culture system (2) comprising at least one growth section (3) which is loaded with living cell fluorescent dyes, and
(iia) measuring the average fluorescence intensity in at least a part of the at least one growth section (3) of said cell culture system at a first time point and at a second time point during culturing of said cell culture system, and comparing the average fluorescence intensity at the second time point with the average fluorescence intensity at the first time point, and/or
(iib) measuring the average fluorescence intensity in at least a part of the at least one growth section (3) of said cell culture system at a time point during culturing of said cell culture system, and comparing the average fluorescence intensity at said time point with the average fluorescence intensity of at least one control cell culture system.

8. The method of claim 7, wherein
(i) a decrease of the average fluorescence intensity at the second time point when compared to the average fluorescence intensity at the first time point indicates that the vitality of said cell culture system (2) is decreased, or
(ii) a retention of the average fluorescence intensity at the second time point when compared to the average fluorescence intensity at the first time point indicates that the vitality of said cell culture system (2) is maintained.

9. The method of claim 7 or 8, wherein
(i) an increase of the average fluorescence intensity at said time point compared to the average fluorescence intensity of the control cell culture system indicates that said cell culture system (2) is vital, or
(ii) the comparability of the average fluorescence intensity at said time point with the average fluorescence intensity of the control cell culture system indicates that said cell culture system (2) is not vital.

10. The method of claims 1 to 9, wherein the at least one growth section (3) comprises a multilayer cell assembly, a tissue, an organoid, or an organ.

## Patentansprüche

1. In-vitro-Verfahren zum Bestimmen und/oder Überwachen mindestens einer Bedingung von/in einem dreidimensionalen Zellkultursystem (2), das mindestens einen Wachstumsabschnitt (3) umfasst, wobei die mindestens eine Bedingung eine physiologische Bedingung umfasst und ferner eine Bedingung umfasst ausgewählt aus der Gruppe bestehend aus:
(i) Vitalität, und
(ii) Metabolismusstatus,
wobei die physiologische Bedingung unter Verwendung von Erythrozyten als Detektoren für diese Bedingung durch Bestimmen der Flussrate, der physiologischen Osmolalität und/oder des Sauerstoffverbrauchs in dem dreidimensionalen Zellkultursystem (2) bestimmt wird, die Vitalität durch Messen von lebenden Zell-Fluoreszenzfarbstoffen bestimmt und/oder überwacht wird, und der Metabolismusstatus durch Messen der Autofluoreszenz von NADH und/oder FAD, und/oder durch Bestimmen des NADH/NAD⁺ und/oder NADH/FAD-Verhältnisses bestimmt und/oder überwacht wird.

2. Verfahren nach Anspruch 1, wobei das Verfahren zur Bestimmung der Flussrate in dem dreidimensionalen Zellkultursystem (2) unter Verwendung von Erythrozyten als Detektoren die folgenden Schritte umfasst:
(i) Bereitstellen eines perfundierbaren dreidimensionalen Zellkultursystems (2), das mindestens einen Wachstumsabschnitt (3) umfasst,
(ii) Aufnehmen von Bildern von Erythrozyten in besagtem Zellkultursystem zu mindestens zwei verschiedenen Zeitpunkten,
(iii) Auswählen mindestens eines Erythrozyten und Bestimmen der Position des mindestens einen Erythrozyten auf den mindestens zwei Bildern,
(iv) Zuweisen eines Bewegungsvektors zu dem mindestens einen Erythrozyten, der die Positionsänderung des mindestens einen Erythrozyten auf den mindestens zwei Bildern repräsentiert, und
(v) Bestimmen der Fließgeschwindigkeit in dem Zellkultursystem auf der Grundlage des Bewegungsvektors.

3. Verfahren nach den Ansprüchen 1 bis 2, wobei das Verfahren zur Bestimmung der physiologischen Osmolalität in dem dreidimensionalen Zellkultursystem (2) unter Verwendung von Erythrozyten als Detektoren die folgenden Schritte umfasst:
(i) Bereitstellen des dreidimensionalen Zellkultursystems (2), das mindestens einen Wachstumsabschnitt (3) umfasst, und
(ii) Bestimmen der Form von mindestens einem Erythrozyten in besagtem Zellkultursystem zu einem Zeitpunkt während der Kultivierung des besagten Zellkultursystems.

4. Verfahren nach Anspruch 3, wobei
(i) eine sphärische Form des mindestens einen Erythrozyten anzeigt, dass die physiologische Osmolalität vermindert ist (hypotonische Bedingung), oder
(ii) eine zackige Form des mindestens einen Erythrozyten anzeigt, dass die physiologische Osmolalität erhöht ist (hypertonische Bedingung), oder
(iii) eine bikonkave Form des mindestens einen Erythrozyten anzeigt, dass die physiologische Osmolalität stabil ist (isotonische Bedingung).

5. Verfahren nach den Ansprüchen 1 bis 4, wobei das Verfahren zur Bestimmung des Sauerstoffverbrauchs in dem dreidimensionalen Zellkultursystem (2) unter Verwendung von Erythrozyten als Detektoren die folgenden Schritte umfasst:
(i) Bereitstellen des dreidimensionalen Zellkultursystems (2) umfassend mindestens einen Wachstumsabschnitt (3),
(ii) Bestimmen einer durchschnittlichen Hämoglobinsättigung von Erythrozyten an einer ersten Position und an einer zweiten Position in besagtem Zellkultursystem, und
(iii) Berechnen des Sauerstoffverbrauchs in besagtem Zellkultursystem auf der Basis der durchschnittlichen Hämoglobinsättigung an den verschiedenen Positionen.

6. Verfahren nach den Ansprüchen 3 bis 5, wobei das dreidimensionale Zellkultursystem (2) perfusionsfähig ist.

7. Verfahren nach Anspruch 1, wobei das Verfahren zum Bestimmen und/oder Überwachen der Vitalität des dreidimensionalen Zellkultursystems (2) durch Messen von lebenden Zell-Fluoreszenzfarbstoffen die folgenden Schritte umfasst:
(i) Bereitstellen des dreidimensionalen Zellkultursystems (2) umfassend mindestens einen Wachstumsabschnitt (3), der mit lebenden Zell-Fluoreszenzfarbstoffen beladen ist, und
(iia) Messen der durchschnittlichen Fluoreszenzintensität in mindestens einem Teil des mindestens einen Wachstumsabschnitts (3) des besagten Zellkultursystems zu einem ersten Zeitpunkt und zu einem zweiten Zeitpunkt während der Kultivierung des besagten Zellkultursystems, und Vergleichen der durchschnittlichen Fluoreszenzintensität zum zweiten Zeitpunkt mit der durchschnittlichen Fluoreszenzintensität zum ersten Zeitpunkt, und/oder
(iib) Messen der durchschnittlichen Fluoreszenzintensität in mindestens einem Teil des mindestens einen Wachstumsabschnitts (3) des besagten Zellkultursystems zu einem Zeitpunkt während der Kultivierung des besagten Zellkultursystems und Vergleichen der durchschnittlichen Fluoreszenzintensität zu besagtem Zeitpunkt mit der durchschnittlichen Fluoreszenzintensität von mindestens einem Kontrollzellkultursystem.

8. Verfahren nach Anspruch 7, wobei
(i) eine Abnahme der durchschnittlichen Fluoreszenzintensität zum zweiten Zeitpunkt im Vergleich zur durchschnittlichen Fluoreszenzintensität zum ersten Zeitpunkt anzeigt, dass die Vitalität des besagten Zellkultursystems (2) erniedrigt ist, oder
(ii) eine Retention der durchschnittlichen Fluoreszenzintensität zum zweiten Zeitpunkt im Vergleich zur durchschnittlichen Fluoreszenzintensität zum ersten Zeitpunkt anzeigt, dass die Vitalität des Zellkultursystems (2) erhalten wird.

9. Verfahren nach Anspruch 7 oder 8, wobei
(i) ein Anstieg der durchschnittlichen Fluoreszenzintensität zu besagtem Zeitpunkt im Vergleich zur durchschnittlichen Fluoreszenzintensität des Kontrollzellkultursystems anzeigt, dass das Zellkultursystem (2) vital ist, oder
(ii) die Vergleichbarkeit der durchschnittlichen Fluoreszenzintensität zu besagtem Zeitpunkt mit der durchschnittlichen Fluoreszenzintensität des Kontrollzellkultursystems anzeigt, dass das Zellkultursystem (2) nicht vital ist.

10. Verfahren nach den Ansprüchen 1 bis 9, wobei der mindestens eine Wachstumsabschnitt (3) eine Mehrschichtzellanordnung, ein Gewebe, ein Organoid oder ein Organ umfasst.

## Revendications

1. Procédé *in vitro* pour la détermination et/ou le suivi d'au moins un état d'/dans un système de culture cellulaire tridimensionnel (2) comprenant au mois une section de croissance (3), où le au moins un état comprend un état physiologique et comprend en outre un état choisi dans le groupe consistant en
(i) vitalité, et
(ii) état du métabolisme,
où l'état physiologique est déterminé au moyen d'érythrocytes comme détecteurs pour ledit état par la détermination de la vitesse d'écoulement, de l'osmolalité physiologique, et/ou de la consommation d'oxygène dans le système de culture cellulaire tridimensionnel (2), la vitalité est déterminée et/ou suivie par la mesure de colorants fluorescents de cellules vivantes, et l'état du métabolisme est déterminé et/ou suivi par la mesure de l'autofluorescence de NADH et/ou de FAD, et/ou par détermination du rapport NADH/NAD⁺ et/ou NADH/FAD.

2. Procédé selon la revendication 1, dans lequel le procédé pour la determination de la vitesse d'écoulement dans le système de culture cellulaire tridimensionnel (2) au moyen d'érythrocytes comme détecteurs comprend les étapes de:
(i) fourniture d'un système de culture cellulaire tridimensionnel perfusable (2) comprenant au moins une section de croissance (3),
(ii) prise de vues d'érythrocytes dans ledit système de culture cellulaire au moins à deux instants différents,
(iii) sélection d'au moins un érythrocyte et détermination de la position du au moins un érythrocyte sur les au mois deux prises de vues,
(iv) l'attribution d'un vecteur de déplacement au au moins un érythrocyte, représentant la modification de position dudit au moins un érythrocyte sur les au moins deux prises de vues, et
(v) détermination de la vitesse d'écoulement dans ledit système de culture cellulaire sur la base du vecteur de déplacement.

3. Procédé selon les revendications 1 ou 2, où le procédé pour la détermination de l'osmolalité physiologique dans le système de culture cellulaire tridimensionnel (2) utilisant des érythrocytes comme détecteurs comprend les étapes de:
(i) fourniture du système de culture cellulaire tridimensionnel (2) comprenant au moins une section de croissance (3), et
(ii) détermination de la forme d'au moins un érythrocyte dans ledit système de culture cellulaire à un instant donné pendant la culture dudit système de culture cellulaire.

4. Procédé selon la revendication 3, où
(i) une forme sphérique du au moins un érythrocyte indique que l'osmolalité physiologique est abaissée (état hypotonique), ou
(ii) une forme crénelée du au moins un érythrocyte indique que l'osmolalité physiologique est augmentée (état hypertonique), ou
(iii) une forme biconcave du au moins un érythrocyte indique que l'osmolalité physiologique est stable (état isotonique).

5. Procédé selon les revendications 1 à 4, où le procédé pour la détermination de la consommation d'oxygène dans le système de culture cellulaire tridimensionnel (2) utilisant des érythrocytes comme détecteurs comprend les étapes de:
(i) fourniture du système de culture cellulaire tridimensionnel (2) comprenant au moins une section de croissance (3),
(ii) détermination d'une saturation moyenne en hémoglobine des érythrocytes sur une première position et sur une deuxième position dans ledit système de culture cellulaire, et
(iii) calcul de la consommation d'oxygène dans ledit système de culture cellulaire sur la base de la saturation en hémoglobine moyenne sur les différentes positions.

6. Procédé selon les revendications 3 ou 5, où le système de culture cellulaire tridimensionnel (2) est perfusable.

7. Procédé selon la revendication 1, où le procédé pour la détermination et/ou le suivi de la vitalité du système de culture cellulaire tridimensionnel (2) par mesure de colorants fluorescents de cellules vivantes comprend les étapes de:
(i) fourniture du système de culture cellulaire tridimensionnel (2) comprenant au moins une section de croissance (3) qui est chargée avec des colorants fluorescents de cellules vivantes, et
(iia) mesure de l'intensité de fluorescence moyenne dans au moins une partie de la au moins une section de croissance (3) dudit système de culture cellulaire à un premier instant et à un deuxième instant pendant la culture du système de culture cellulaire, et la comparaison de l'intensité de fluorescence moyenne au deuxième instant avec l'intensité de fluorescence moyenne au premier instant, et/ou
(iib) mesure de l'intensité de fluorescence moyenne dans au moins une partie de la au moins une section de croissance (3) dudit système de culture cellulaire à un instant pendant la culture dudit système de culture cellulaire, et comparaison de l'intensité de fluorescence moyenne audit instant avec l'intensité de fluorescence moyenne du au moins un système de culture cellulaire de référence.

8. Procédé selon la revendication 7, où
(i) une diminution de l'intensité de fluorescence moyenne au deuxième instant lors de la comparaison à l'intensité de fluorescence moyenne au premier instant indique que la vitalité dudit système de culture cellulaire (2) est diminuée, ou
(ii) une rétention de l'intensité de fluorescence moyenne au deuxième instant lorsqu'elle est comparée à l'intensité de fluorescence moyenne au premier instant indique que la vitalité dudit système de culture cellulaire (2) est maintenue.

9. Procédé selon la revendication 7 ou 8, où
(i) une augmentation de l'intensité de fluorescence moyenne audit instant par rapport à l'intensité de fluorescence moyenne du système de culture cellulaire tridimensionnel de référence indique que ledit système de culture cellulaire (2) est vital, ou
(ii) la comparabilité de l'intensité de fluorescence moyenne audit instant avec l'intensité de fluorescence moyenne du système de culture cellulaire de référence indique que ledit système de culture cellulaire (2) n'est pas vital.

10. Procédé selon les revendications 1 à 9, où la au moins une section de croissance (3) comprend un ensemble de cellules multicouche, un tissu, un organoïde, ou un organe.
